(19) Europäisches Patentamt
European Patent Office
Office européen des brevets

(11) EP 1 033 965 B1

(12) # EUROPÄISCHE PATENTSCHRIFT

(45) Veröffentlichungstag und Bekanntmachung des Hinweises auf die Patenterteilung:
**24.11.2004 Patentblatt 2004/48**

(21) Anmeldenummer: **99911716.1**

(22) Anmeldetag: **26.02.1999**

(51) Int Cl.$^7$: **A61K 7/13**

(86) Internationale Anmeldenummer:
**PCT/EP1999/001266**

(87) Internationale Veröffentlichungsnummer:
**WO 1999/059529 (25.11.1999 Gazette 1999/47)**

(54) **MITTEL UND VERFAHREN ZUR ERZEUGUNG VON SEMIPERMANENTEN FÄRBUNGEN AUF KERATINFASERN**

AGENTS AND METHOD FOR PRODUCING SEMI-PERMANENT COLORATIONS OF KERATIN FIBERS

PRODUITS ET PROCEDE DESTINES A DES COLORATIONS SEMI-PERMANENTES DE FIBRES KERATINIQUES

(84) Benannte Vertragsstaaten:
**AT CH DE ES FR GB IT LI**

(30) Priorität: **16.05.1998 DE 19822198**

(43) Veröffentlichungstag der Anmeldung:
**13.09.2000 Patentblatt 2000/37**

(73) Patentinhaber: **Wella Aktiengesellschaft
64274 Darmstadt (DE)**

(72) Erfinder:
- **GÖTTEL, Otto
CH-1723 Marly (CH)**
- **PIRRELLO, Aline
CH-1762 Givisiez (CH)**

(56) Entgegenhaltungen:
**EP-A- 0 833 314** **WO-A-99/44569**

- **BERLIN ET AL.: "Methoden zur Herstellung von Cyaninen (polymethinen)" HOUBEN-WEYL, Bd. 5, Nr. 1d, Seiten 227-299, XP002117941 in der Anmeldung erwähnt**
- **HENSLEY ET AL.: "POLYMETHINE DYES" KIRK-OTHMER ENCYCL. CHEM. TECHNOL. SECOND EDITION, Bd. 16, 1968, Seiten 282-291, XP002117942**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 033 965 B1

Printed by Jouve, 75001 PARIS (FR)

## Beschreibung

Die vorliegende Erfindung betrifft nicht-oxidative Färbemittel für Keratinfasern, wie zum Beispiel Haare oder Wolle, auf der Basis einer Kombination von natürlichen oder synthetischen nicht-oxidativen Farbstoffen (sogenannten "Direktziehern") und Polymethinfarbstoffen sowie ein Verfahren zum Färben von Keratinfasem.

Die Färbung von Haaren ist in der heutigen Zeit den unterschiedlichsten Trends unterworfen. Während früher Haare vor allem gefärbt wurden, um Grauanteile zu überdecken, besteht heute immer mehr das Verlangen nach der Integration der Haarfarbe in die aktuelle Mode sowie als Ausdruck der Persönlichkeit.

Zur Färbung von Haaren stehen nach wie vor zwei etablierte Methoden zur Verfügung. Zum einen ist dies die oxidative Haarfärbung, die zu einem sehr dauerhaften Färbeergebnis führt. Zum anderen besteht die Möglichkeit, die Haare mit nicht-oxidativen, direktziehende Farbstoffe enthaltenden Färbemitteln (häufig als Tönungsmittel bezeichnet) zu färben. Obwohl die dafür verwendeten Farbstoffe neben der färberischen Leistung darauf optimiert sind, möglichst lange im Haar zu verbleiben, werden mit jedem Waschvorgang die Tönungsergebnisse graduell schwächer, so daß je nach verwendetem Produkt und Haartyp solche Färbungen in der Regel maximal 10 Haarwäschen überdauem.

Es bestand daher die Aufgabe, nicht-oxidative Färbemittel zur Verfügung zu stellen, die neben guten färberischen Eigenschaften eine verbesserte Haltbarkeit der Färbung, insbesondere in Bezug auf die Auswaschstabilität, aufweisen.

Überraschenderweise wurde nunmehr gefunden, daß Färbemittel auf der Basis einer Kombination aus natürlichen oder synthetischen nicht-oxidativen Farbstoffen und Polymethinfarbstoffen (im folgenden auch "Oxonolfarbstoffe" genannt) eine hervorragende und vor allem äußerst dauerhafte Färbung von Keratinfasem ermöglichen.

Oxonolfarbstoffe sind bereits seit langem bekannt und man findet in der Literatur eine sehr breite Palette von Substitutionsmustern. Einen Überblick über diese Farbstoffe findet man beispielsweise in Houben-Weyl 5/1 d, 4. Auflage (1954), Seite 227 ff. Einige dieser Oxonolfarbstoffe sind zudem im Handel erhältlich.

Mit dem erfindungsgemäßen Färbemittel lassen sich Nuancen im abgewandelten Naturton, vor allem aber im modischen Bereich erzielen. Darüber hinaus ist es möglich, neben den erwähnten Tönen eine Reihe von brillanten Farbreflexen vor allem im hellroten bis blauen Bereich zu erzielen. Bedingt durch die sehr hohe Farbstärke der Farbstoffe und das gute Aufziehverhalten kann in hervorragender Weise der ursprüngliche Farbton der Faser überdeckt werden. Damit kann auch dem eingangs erwähnten Wunsch nach der Integration der Haarfarbe in die Mode sowie als Ausdruck der Persönlichkeit voll entsprochen werden.

Gegenstand der vorliegenden Anmeldung ist daher ein Mittel zur Färbung von Keratinfasern, wie zum Beispiel Pelzen, Federn, Wolle, Haaren, insbesondere menschlichen Haaren, welches dadurch gekennzeichnet ist, daß es eine Kombination aus (a) mindestens einem natürlichen oder synthetischen nicht-oxidativen Farbstoff, welcher ausgewählt ist aus natürlichen Farbstoffen oder Nitrofarbstoffen, Azofarbstoffen, Chinonfarbstoffen, Triphenylmethanfarbstoffen und basischen oder sauren Farbstoffen, und (b) mindestens einem Polymethinfarbstoff der allgemeinen Formeln (I) bis (III) oder deren physiologisch verträglichen Salze, enthält, dargestellt in ihres Säureform und in einer ihrer möglichen tautomeren Formen,

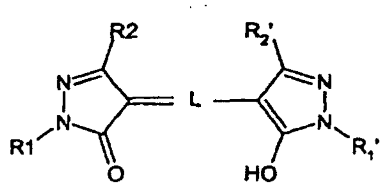

(I)

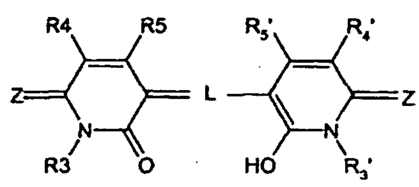

(II)

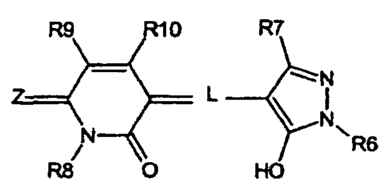

(III)

worin **R1, R1' und R6** Wasserstoff, eine geradkettige oder verzweigte C1- bis C8- Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest oder einen mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierten Phenylrest, einen Benzylrest oder einen mit einem oder mehreren Halogenatomen, mit einer C1- bis C4-Alkylgruppe, einer Hydroxygruppe, einer Methoxygruppe, einer Carboxygruppe, einer Nitrogruppe oder einer Aminogruppe substituierten Benzylrest oder einen fünfgliedrigen oder sechsgliedrigen gesättigten oder ungesättigten Heterocyclus bedeuten, wobei **R1 und R1'** gleich oder verschieden sein können,und

**R2, R2' und R7** Wasserstoff, eine verzweigte oder unverzweigte C1- bis C6- Alkylgruppe, einen Phenylrest, eine Aminogruppe, die zusätzlich acyliert oder sulfonyliert sein kann, eine Acetylgruppe, eine Methoxygruppe, eine Carbonsäuregruppe, die mit einem geradkettigen oder verzweigten C1- bis C8-Alkohol oder einem Ethylenglykolmonomethylether oder einem Ethylenglykolmonoethylether verestert sein kann, eine Carbonsäureamidgruppe, eine Carbonsäureanilidgruppe, eine 2-Amino-2-oxyethylgruppe oder eine Nitrilgruppe bedeuten, wobei **R2 und R2'** gleich oder verschieden sein können, und

**R3, R3' und R8** Wasserstoff, eine geradkettige oder verzweigte C1- bis C11-Alkylgruppe, eine geradkettige oder verzweigte C1- bis C11-Monohydroxyalkylgruppe, eine geradkettige oder verzweigte C1- bis C11-Dihydroxyalkylgruppe, eine geradkettige oder verzweigte C1- bis C11-Alkoxyalkylgruppe, eine geradkettige oder verzweigte C1- bis C11-Monoalkylaminogruppe, eine Aminogruppe der Formel $(CH_2)_x$-NR11R12 (mit x gleich einer ganzen Zahl von 0 bis 3 und R11 und R12 unabhängig voneinander gleich einer C1- bis C3-Alkylgruppe), eine C2- bis C4-Sulfoalkylgruppe oder Carboxyalkylgruppe, eine Phenylgruppe oder eine mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierte Phenylgruppe, eine Benzylgruppe oder eine mit einem oder mehreren Halogenatomen, mit einer C1- bis C4-Alkylgruppe, einer Hydroxygruppe, einer Methoxygruppe, einer Nitrogruppe oder einer Aminogruppe substituierte Benzylgruppe, eine Phenylethylgruppe, ein fünfgliedriger oder sechsgliedriger aromatischer oder nicht-aromatischer Heterocyclus, der direkt oder über eine Methylengruppe gebunden sein kann, eine Pyrrolidinogruppe , Morpholinogruppe , Piperazinogruppe , Piperidinogruppe oder Pyridino(C2- oder C3-)alkylgruppe oder eine Trialkylammoniumalkylgruppe der Formel R13-$N(R14)_3^+$ (mit R13 gleich einer C1- bis C6-Alkylengruppe und R14 gleich einer Methyl- oder Ethylgruppe, wobei die Gesamtzahl an Kohlenstoffatomen im Molekül gleich 5 bis 9 ist) bedeuten,

wobei **R3 und R3'** gleich oder verschieden sein können, und

**R4, R4' und R9** Wasserstoff, eine Nitrilgruppe, eine Carbonsäureestergruppe, eine Carbonsäureamidgruppe, eine Sulfonsäuregruppe, eine Sulfomethylgruppe, eine Methansulfonylgruppe, einen Pyridiniumrest oder einen Imidazoliumrest bedeuten, wobei **R4 und R4'** gleich oder verschieden sein können, und

**R5, R5' und R10** Wasserstoff, eine C1- bis C4-Alkylgruppe, eine C5- bis C6-Cycloalkylgruppe, eine Phenylgruppe, eine Methoxyphenylgruppe, eine Benzylgruppe, eine Phenylethylgruppe oder eine Carbonsäuregruppe bedeuten, wobei **R5 und R5'** gleich oder verschieden sein können, und

**Z** für Sauerstoff oder einen Rest der Formel $C(CN)_2$, C(CN)COOQ oder $C(COOQ)_2$, worin Q jeweils eine C1- bis C8-Alkylgruppe oder einen Ethylenglykolmono(C3- bis C7-)alkylether darstellt, steht, und

**L** für eine Brückengruppe der allgemeinen Formel

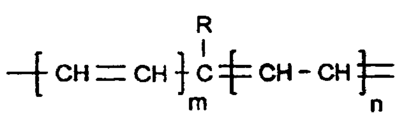

steht, worin **R** ein Wasserstoffatom, eine Phenylgruppe, eine Methylgruppe, eine Carbonamidgruppe oder ein Halogenatom darstellt, und die Indices **m** und **n** jeweils gleich 0, 1 oder 2 sein können, wobei die Summe aus m und n nicht größer als 2 ist.

Besonders bevorzugt sind Farbstoffe der allgemeinen Formeln (I) bis (III) sowie deren Salze, in denen

**R1 und R1'** gleich sind und Wasserstoff, eine geradkettige oder verzweigte C1- bis C4-Alkylgruppe, eine Hydroxyethylgruppe, eine Dihydroxypropylgruppe, eine Methoxyethylgruppe, eine C2- bis C4-Sulfoalkylgruppe, einen Phenylrest oder einen mit einem oder mehreren Halogenatomen, einer oder zwei Sulfonsäuregruppen, einer Methylgruppe oder einer Methoxygruppe substituierten Phenylrest oder einen Benzylrest oder einen mit einem Halogenatom, mit einer Methylgruppe, Hydroxygruppe, Methoxygruppe, Nitrogruppe oder Aminogruppe substituierten Benzylrest bedeuten und

**R2 und R2'** gleich sind und eine Methylgruppe, eine Aminogruppe, die zusätzlich acyliert sein kann, oder eine Carbonsäuregruppe, die mit einem C1- bis C3-Alkohol verestert sein kann, bedeuten und

**R3 und R3'** gleich oder verschieden sind und Wasserstoff, eine geradkettige C1- bis C4-Alkylgruppe, eine Hydroxyethylgruppe, eine Methoxyethylgruppe, eine Methoxypropylgruppe, eine Sulfoethylgruppe, einen Phenylrest oder einen mit einem Halogenatom, einer Sulfonsäuregruppe, einer Methylgruppe oder Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest oder einen fünfgliedrigen oder sechsgliedrigen aromatischen oder nicht-aromatischen Heterocyclus, der direkt oder über eine Methylengruppe gebunden sein kann, eine Aminogruppe, eine C1- bis C4-Monoalkyl-aminogruppe, eine Dialkylaminogruppe mit insgesamt 2 bis 8 Kohlenstoffatomen im Molekül oder eine Trialkylammaniumafkylgruppe der Formel $R13\text{-}N(R14)_3^+$ (mit R13 gleich einer C1- bis C6-Alkylengruppe und R14 gleich einer Methyloder Ethylgruppe, wobei die Gesamtzahl an Kohlenstoffatomen im Molekül gleich 5 bis 9 ist) bedeuten und

**R4 und R4'** gleich sind und Wasserstoff, eine Nitrilgruppe, eine Carbonsäureamidgruppe, eine Sulfonsäuregruppe, eine Sulfomethylgruppe, eine Methansulfonylgruppe, eine Pyridiniumgruppe oder eine Imidazoliumgruppe bedeuten und

**R5 und R5'** gleich sind und Wasserstoff, eine Methylgruppe, eine Nitrilgruppe oder einen substituierten oder unsubstituierten Phenylrest bedeuten und

**R6** Wasserstoff, eine geradkettige oder verzweigte C1- bis C4-Alkylgruppe, eine Hydroxyethylgruppe, eine Dihydroxypropylgruppe, eine Methoxyethylgruppe, eine C2- bis C4-Sulfoalkylgruppe, einen Phenylrest oder einen mit einem oder mehreren Halogenatomen, einer oder zwei Sulfonsäuregruppen, einer Methylgruppe oder einer Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest bedeutet und

R7 eine Methylgruppe, eine Aminogruppe, die zusätzlich acyliert sein kann, eine Carbonsäuregruppe oder eine mit einem C1- bis C3-Alkohol veresterte Carbonsäuregruppe, bedeutet und

R8 Wasserstoff, eine geradkettige C1- bis C4-Alkylgruppe, eine Hydroxyethylgruppe, eine Methoxyethylgruppe, eine Sulfoethylgruppe, einen Phenylrest oder einen mit einem Halogenatom, einer Sulfonsäuregruppe, einer Methylgruppe oder Methoxygruppe substituierten Phenylrest, einen Benzylrest oder einen mit einem Halogenatom, mit einer Methylgruppe, Hydroxygruppe, Methoxygruppe, Nitrogruppe oder Aminogruppe substituierten Benzylrest oder einen fünfgliedrigen oder sechsgliedrigen aromatischen oder nicht-aromatischen Heterocyclus, der direkt oder über eine Methylengruppe gebunden sein kann, eine Aminogruppe, eine C1- bis C4-Monoalkylaminogruppe, eine Dialkylaminogruppe mit insgesamt 2 bis 8 Kohlenstoffatomen im Molekül oder eine Trialkylammoniumalkylgruppe der Formel $R13\text{-}N(R14)_3^+$ (mit R13 gleich einer C1- bis C6-Alkylengruppe und R14 gleich einer Methyloder Ethylgruppe, wobei die Gesamtzahl an Kohlenstoffatomen im Molekül gleich 5 bis 9 ist) bedeutet und

R9 Wasserstoff, eine Nitrilgruppe, eine Carbonsäureamidgruppe, eine Sulfonsäuregruppe, eine Sulfomethylgruppe, eine Pyridiniumgruppe oder eine Imidazoliumgruppe bedeutet und

R10 Wasserstoff, eine Methylgruppe, eine Nitrilgruppe oder einen substituierten oder unsubstituierten Phenylrest bedeutet und

**Z** gleich Sauerstoff oder einer $C(CN)_2$-Gruppe ist und

**L** eine Brückengruppe der allgemeinen Formel

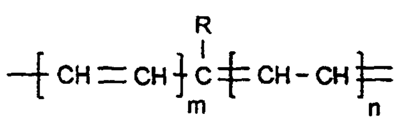

darstellt, worin **R** ein Wasserstoffatom oder eine Methylgruppe darstellt und die Indices **m** und **n** jeweils gleich 0, 1 oder 2 sein können, wobei die Summe aus m und n nicht größer als 2 ist.

Als Beispiele für die vorgenannten bevorzugten Farbstoffe der Formeln (I) bis (III) können die nachfolgend aufgeführten Verbindungen (in jeweils einer ihrer möglichen Tautomeren) genannt werden. In der Säureform geschrieben sind dies:

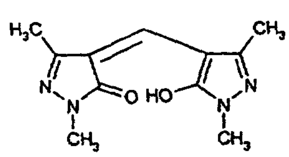

4-(5-Hydroxy-1,3-dimethyl-1H-pyrazol-4-ylmethylen)-2,5-dimethyl-2,4-dihydropyrazol-3-on **(1)**

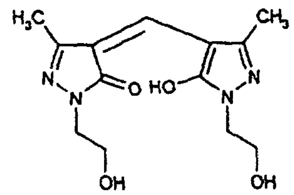

2-(2-Hydroxyethyl)-4-(5-hydroxy-1-(2-hydroxyethyl)-3-methyl-1H-pyrazol-4-ylmethylen)-5-methyl-2,4-dihydropyrazol-3-on **(2)**

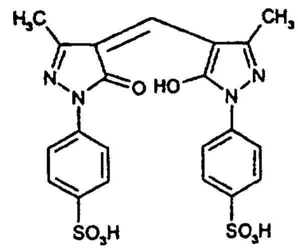

4-(5-Hydroxy-3-methyl-1-(4-sulfophenyl)-1H-pyrazol-4-ylmethylen)-5-methyl-2-(4-sulfophenyl)-2,4-dihydropyrazol-3-on **(3)**

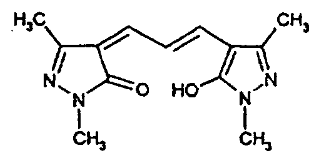

4-(3-(5-Hydroxy-1,3-dimethyl-1H-pyrazol-4-yl)-allyliden)-2,5-dimethyl-2,4-dihydropyrazol-3-on **(4)**

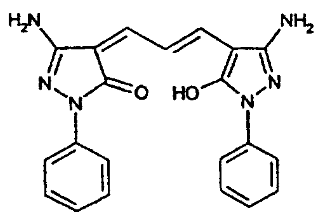

5-Amino-4-(3-(3-amino-5-hydroxy-1-phenyl-1H-pyrazol-4-yl)-allyliden)-2-phenyl-2,4-dihydropyrazol-3-on **(5)**

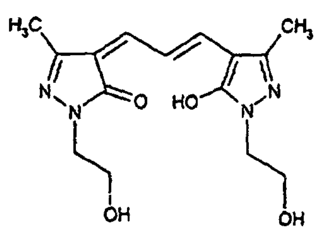

2-(2-Hydroxyethyl)-4-(3-(5-hydroxy-1-(2-hydroxyethyl)-3-methyl-1Hpyrazol-4-yl)-allyliden)-5-methyl-2,4-dihydro-pyrazol-3-on **(6)**

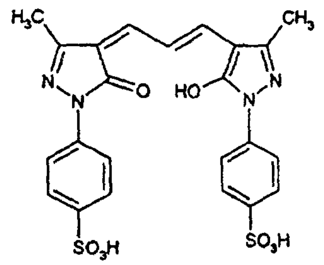

4-(3-(5-Hydroxy-3-methyl-1-(4-sulfophenyl)-1H-pyrazol-4-yl)-allyliden)-5-methyl-2-(4-sulfophenyl)-2,4-dihydro-pyrazol-3-on **(7)**

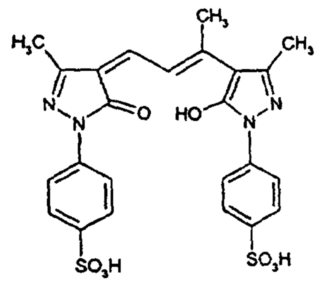

4-(3-(5-Hydroxy-3-methyl-1-(4-sulfophenyl)-1H-pyrazol-4-yl)-but-2-enyliden)-5-methyl-2-(4-sulfophenyl)-2,4-dihydro-pyrazol-3-on **(8)**

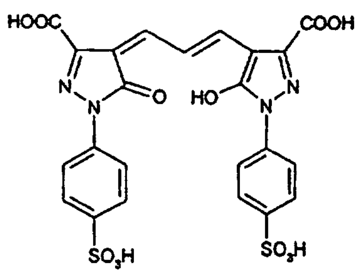

4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure **(9)**

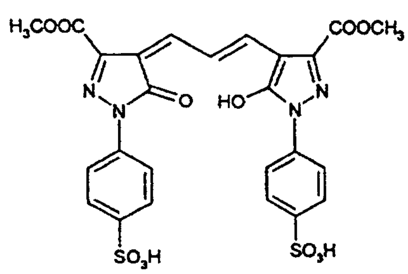

5-Hydroxy-4-(3-(3-methoxycarbonyl-5-oxo-1-(4-sulfophenyl)-1,5-dihydropyrazol-4-yliden)-propenyl)-1-(4-sulfophenyl)-1H-pyrazol-3-carbonsäuremethylester (10)

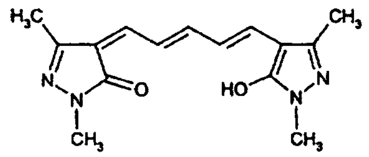

4-(5-(5-Hydroxy-1,3-dimethyl-1H-pyrazol-4-yl)-penta-2,4-dienyliden)-2,5-dimethyl-2,4-dihydropyrazol-3-on **(11)**

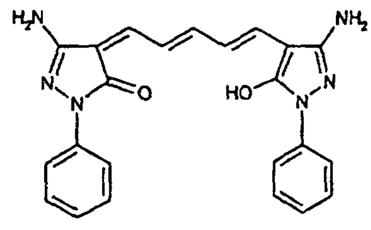

5-Amino-4-(5-(3-amino-5-hydroxy-1-phenyl-1H-pyrazol-4-yl)-penta-2,4-dienyliden)-2-phenyl-2,4-dihydropyrazol-3-on **(12)**

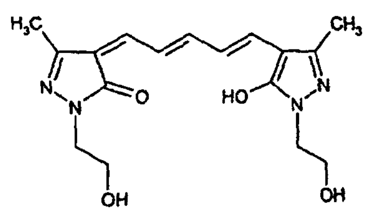

2-(2-Hydroxyethyl)-4-(5-(5-hydroxy-1-(2-hydroxyethyl)-3-methyl-1Hpyrazol-4-yl)-penta-2,4-dienyliden)-5-methyl-2,4-dihydro-pyrazol-3-on **(13)**

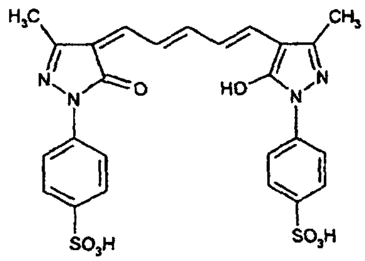

4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on **(14)**

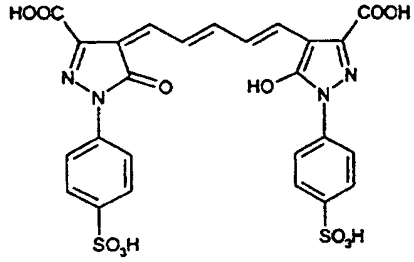

4-(5-(3-Carboxy-5-oxo-1-(4-sulfopheny))-1,5-dihydropyrazol-4-yliden)-penta-1,3-dienyl)-5-hydroxy-1-(4-sulfophenyl)-1H-pyrazol-3-carbonsäure **(15)**

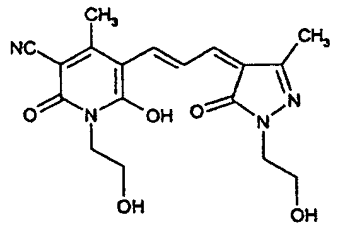

6-Hydroxy-1-(2-hydroxyethyl)-5-(3-(1-(2-hydroxyethyl)-3-methyl-5-oxo-1,5-dihydro-pyrazol-4-yliden)-propenyl)-4-methyl-2-oxo-1,2-dihydro-pyridin-3-carbonitril **(16)**

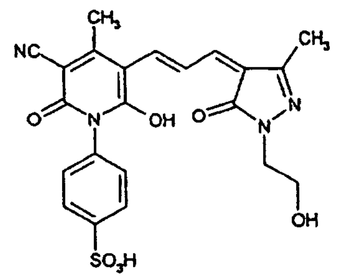

6-Hydroxy-5-(3-(1-(2-hydroxyethyl)-3-methyl-5-oxo-1,5-dihydro-pyrazol-4-yliden)-propenyl)-4-methyl-2-oxo-1-(4-sulfophenyl)-1,2-dihydro-pyridin-3-carbonitril **(17)**

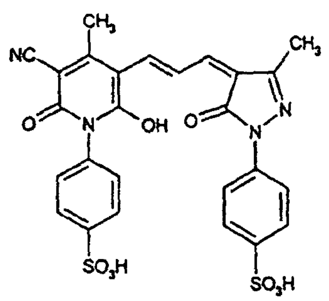

6-Hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril **(18)**

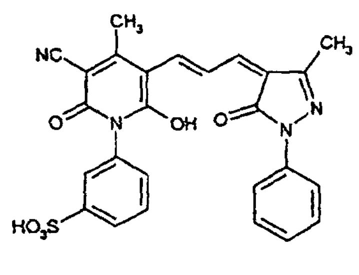

6-Hydroxy-4-methyl-5-(3-(3-methyl-5-oxo-1-phenyl-1,5-dihydropyrazol-4-yliden)-propenyl)-2-oxo-1-(3-sulfophenyl)-1,2-dihydropyridin-3-carbonitril **(19)**

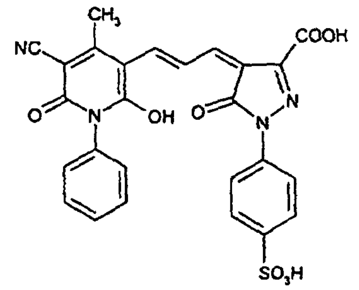

4-(3-(5-Cyano-2-hydroxy-4-methyl-6-oxo-1-phenyl-1,6-dihydropyridin-3-yl)-allyliden)-5-oxo-1-(4-sulfophenyl)-4,5-dihydro-1H-pyrazol-3-carbonsäure **(20)**

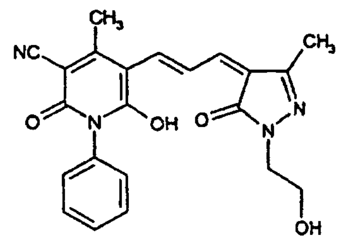

6-Hydroxy-5-(3-(1-(2-hydroxyethyl)-3-methyl-5-oxo-1,5-dihydropyrazol-4-yliden)-propenyl)-4-methyl-2-oxo-1-phenyl-1,2-dihydropyridin-3-carbonitril **(21)**

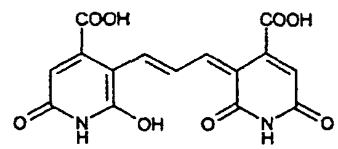

5-(3-(4-Carboxy-2,6-dioxo-1,6-dihydro-2H-pyridin-3-yliden)-propenyl)-6-hydroxy-2-oxo-1,2-dihydropyridin-4-carbonsäure **(22)**

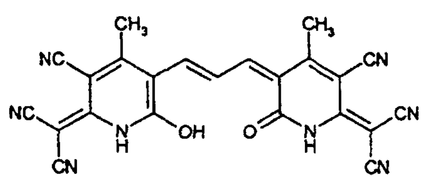

2-(3-Cyano-S-(3-(5-cyano-6-dicyanomethylen-4-methyl-2-oxo-1,6-dihydro-2H-pyridin-3-yliden)-propenyl)-6-hydroxy-4-methyl-1H-pyridin-2-yliden)-malononitril **(23)**

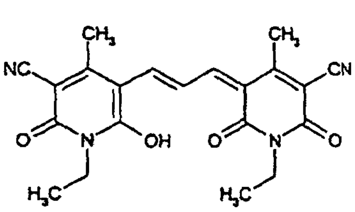

5-(3-(5-Cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24)**

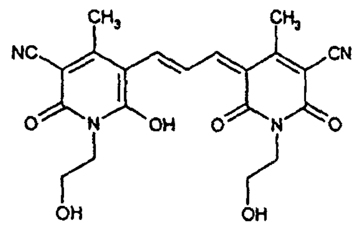

5-(3-(5-Cyano-1-(2-hydroxyethyl)-4-methyl-2,6-dioxo-1,6-dihydro-2Hpyridin-3-yliden)-propenyl)-6-hydroxy-1-(2-hydroxyethyl)-4-methyl-2-oxo-1,2-dihydropyridin-3-carbonitril **(25)**

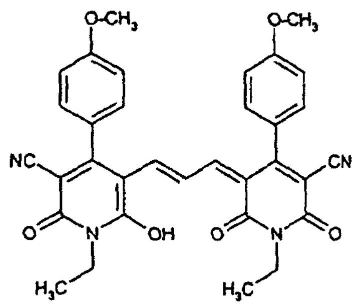

5-(3-(5-Cyano-1-ethyl-4-(4-methoxyphenyl)-2,6-dioxo-1,6-dihydro-2Hpyridin-3-yliden)-propenyl)-1-ethyl-6-hydroxy-4-(4-methoxyphenyl)-2-oxo-1,2-dihydropyridin-3-carbonitril **(26)**

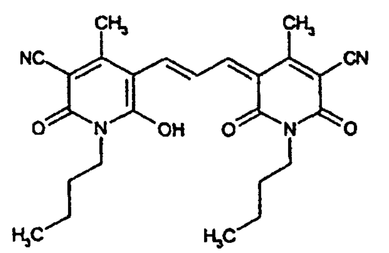

1-Butyl-5-(3-(1-butyl-5-cyano-4-methyl-2,6-dioxo-1,6-dihydro-2H-pyridin-3-yliden)-propenyl)-6-hydroxy-4-methyl-2-oxo-1,2-dihydropyridin-3-carbonitril **(27)**

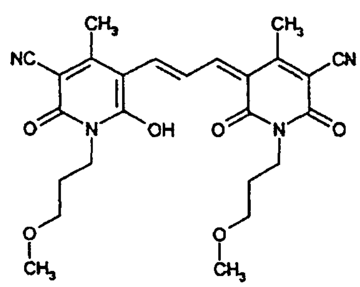

5-(3-(5-Cyano-1-(3-methoxy-propyl)-4-methyl-2,6-dioxo-1,6-dihydro-2Hpyridin-3-yliden)-propenyl)-6-hydroxy-1-(3-methoxy-propyl)-4-methyl-2-oxo-1,2-dihydropyridin-3-carbonitril **(28)**

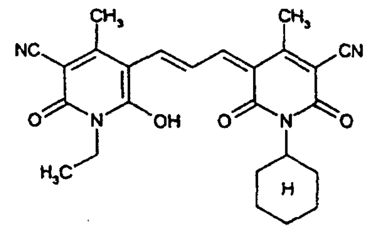

5-(3-(5-Cyano-1-cyclohexyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(29)**

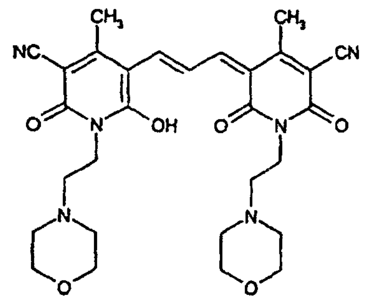

5-(3-(5-Cyano-4-methyl-1-(2-morpholin-4-yl-ethyl)-2,6-dioxo-1,6-dihydro-2H-pyridin-3-yliden)-propenyl)-6-hydroxy-4-methyl-1-(2-morpholin-4-ylethyl)-2-oxo-1,2-dihydropyridin-3-carbonitril **(30)**

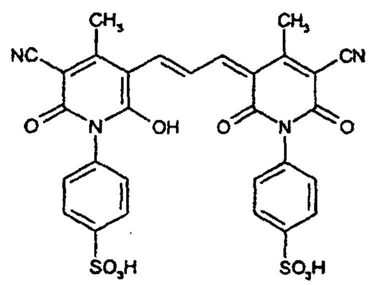

5-(3-(5-Cyano-4-methyl-2,6-dioxo-1-(4-sulfophenyl)-1,6-dihydro-2Hpyridin-3-yliden)-propenyl)-6-hydroxy-4-methyl-2-oxo-1-(4-sulfophenyl)-1,2-dihydropyridin-3-carbonitril **(31)**

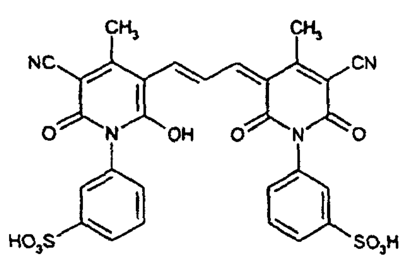

5-(3-(5-Cyano-4-methyl-2,6-dioxo-1-(3-sulfophenyl)-1,6-dihydro-2Hpyridin-3-yliden)-propenyl)-6-hydroxy-4-methyl-2-oxo-1-(3-sulfophenyl)-1,2-dihydropyridin-3-carbonitril **(32)**

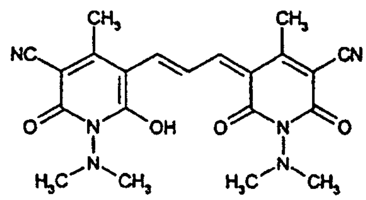

5-(3-(5-Cyano-1-dimethylamino-4-methyl-2,6-dioxo-1,6-dihydro-2Hpyridin-3-yliden)-propenyl)-1-dimethylamino-6-hydroxy-4-methyl-2-oxo-1,2-dihydropyridin-3-carbonitril **(33)**

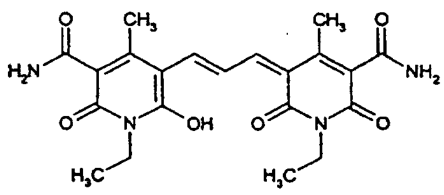

5-(3-(5-Carbamoyl-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonsäureamid **(34)**

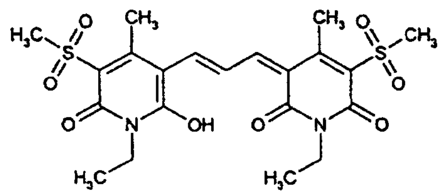

1-Ethyl-3-(3-(1-ethyl-2-hydroxy-5-methansulfonyl-4-methyl-6-oxo-1,6-dihydro-pyridin-3-yl)-allyliden)-5-methansulfonyl-4-methyl-3H-pyridin-2,6-dion **(35)**

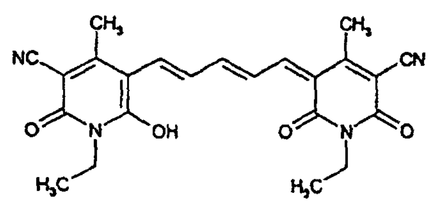

5-(5-(5-Cyano-1-ethyl-4-methyl-2,6-dioxo-1,6-dihydro-2H-pyridin-3-yliden)-penta-1,3-dienyl)-1-ethyl-6-hydroxy-4-methyl-2-oxo-1,2-dihydropyridin-3-carbonitril **(36)**

Unter den vorgenannten Farbstoffen sind insbesondere Verbindungen der Formel (I) bis (III) bevorzugt, bei denen

**R1 und R1'** gleich sind und eine Phenylgruppe, eine Sulfophenylgruppe, eine Methylgruppe oder eine Hydroxyethylgruppe bedeuten;

**R2 und R2'** gleich sind und Wasserstoff, eine Methylgruppe, eine Carbonsäuregruppe, eine Carbonsäureestergruppe oder eine Aminogruppe bedeuten;

**R3 und R3'** unabhängig voneinander Wasserstoff oder eine geradkettige C1-C4-Alkylgruppe, eine Methoxyethylgruppe, eine Hydroxyethylgruppe, eine Sulfoethylgruppe, eine Phenylgruppe oder eine Sulfophenylgruppe bedeuten;

**R4 und R4'** gleich sind und Wasserstoff, eine Nitrilgruppe oder eine Carbonsäureamidgruppe bedeuten;

**R5 und R5'** gleich sind und eine Methylgruppe oder eine Carbonsäuregruppe bedeuten;

**R6** eine Phenylgruppe, eine Sulfophenylgruppe, eine Methylgruppe oder eine Hydroxyethylgruppe bedeutet;

**R7** Wasserstoff, eine Methylgruppe, eine Carbonsäuregruppe, eine Carbonsäureestergruppe oder eine Aminogruppe

bedeutet;
**R8** Wasserstoff oder eine geradkettige C1-C4-Alkylgruppe, eine Methoxyethylgruppe, eine Hydroxyethylgruppe, eine Sulfoethylgruppe,
eine Phenylgruppe oder eine Sulfophenylgruppe bedeutet;
**R9** eine Nitrilgruppe bedeutet;
**R10** eine Methylgruppe oder eine Carbonsäuregruppe bedeutet;
**Z** gleich Sauerstoff ist;
**R** einem Wasserstoffatom oder einer Methylgruppe entspricht und
die Indices **m** und **n** jeweils gleich 0, 1 oder 2 sein können, wobei die Summe aus **m** und **n** maximal den Wert 2 ergibt.

Bei Farbstoffen mit ausgeprägtem anionogenen Charakter (dies gilt vor allem für die Farbstoffe, die Säurefunktionen wie Sulfonsäure- oder Carbonsäuregruppen enthalten) können diese Farbstoffe auch in Form der physiologisch verträglichen Salze eingesetzt werden. Insbesondere können die folgenden Verbindungen genannt werden:

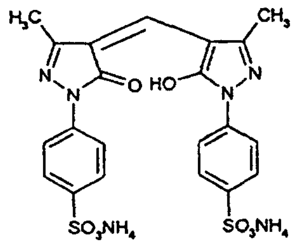

Diammonium-4-(5-hydroxy-3-methyl-1-(4-sulfophenyl)-1H-pyrazol-4-ylmethylen)-5-methyl-2-(4-sulfophenyl)-2,4-dihydro-pyrazol-3-on **(3a)**

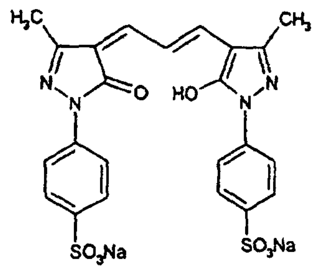

Dinatrium-4-(3-(5-hydroxy-3-methyl-1-(4-sulfophenyl)-1H-pyrazol-4-yl)-allyliden)-5-methyl-2-(4-sulfophenyl)-2,4-dihydro-pyrazol-3-on **(7a)**

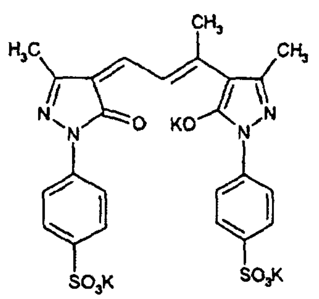

Trikalium-4-(3-(5-hydroxy-3-methyl-1-(4-sulfophenyl)-1H-pyrazol-4-yl)-but-2-enyliden)-5-methyl-2-(4-sulfophenyl)-2,4-dihydro-pyrazol-3-on **(8a)**

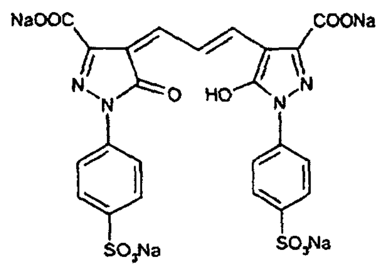

Tetranatrium-4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäu re **(9a)**

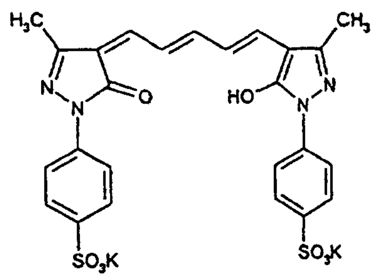

Dikalium-4-(5-(5-Hydroxy-3-methyl-1-(4-sulfophenyl)-1H-pyrazol-4-yl)-penta-2,4-dienyliden}-5-methyl-2-(4-sulfophenyl)-2,4-dihydropyrazol-3-on **(14a)**

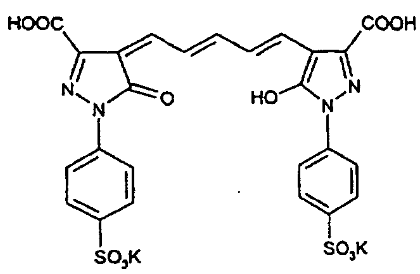

Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)**

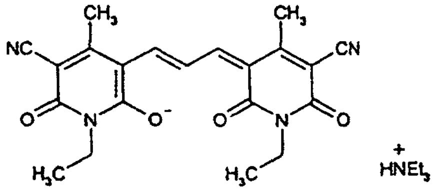

Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)**

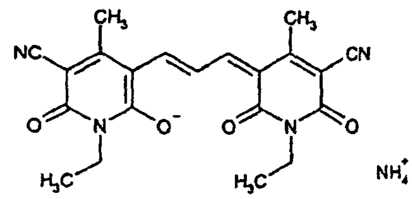

Ammonium-5-(3-(5-cyano-1-ethyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitril **(24b)**

Der Gesamtgehalt an den Farbstoffen der Formeln (I) bis (III), beträgt in dem erfindungsgemäßen Mittel zur Färbung von Keratinfasem 0,01 bis 5 Gewichtsprozent, vorzugsweise 0,5 bis 4 Gewichtsprozent.

Das erfindungsgemäße Färbemittel enthält weiterhin mindestens einen natürlichen oder synthetischen nicht-oxidativen Farbstoff. Als synthetische nicht-oxidative Farbstoffe können beispielsweise Nitrofarbstoffe, Azofarbstoffe, Triphenylmethanfarbstoffe, Chinonfarbstoffe und Anthrachinonfarbstoffe oder saure Farbstoffe genannt werden, insbesondere

1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxyethyl) amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1-Amino-4-[(2-hydroxyethyl)amino]-2-nitrobenzol (HC Red No. 7), 2-Amino-4,6-dinitrophenol, 4-Amino-2-nitro-diphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-ethylamino-4-nitrophenol, 2-Amino-6-chlor-4-nitrophenol-Hydrochlorid, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)-amino]-5-nitrobenzol (HC Yellow No. 5), 1-(2-Hydroxyethoxy)-2-[(2-hydroxyethyl)amino]-5-nitrobenzol (HC Yellow No. 4), 1-[(2-Hydroxyethyl)-amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Amino-ethyl)amino]-1-methoxy-4-nitrobenzol-hydrochlorid (HC Yellow No.9), 1-[(2-Ureidoethyl) amino]-4-nitrobenzol, 4-[(2,3-Dihydroxypropyl)amino]-3-nitro-1-trifluormethyl-benzol (HC Yellow No. 6), 1-Chlor-2,4-bis[(2-hydroxy-ethyl)amino)-5-nitrobenzol (HC Yellow No. 10), 4-[(2-Hydroxyethyl)-amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl) amino]-3-nitro-benzamid (HC Yellow No. 15), 1,4-Di[(2,3-dihydroxypropyl)amino]-9,10-anthrachinon, 1-[(2-Hydroxyethyl)amino]-4-methylamino-9,10-anthrachinon (Cl61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylarnino-9,10-anthrachinon (HC Blue No. 8), 1-[(3-Aminopropyl)-amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69),1-[Di(2-hydroxy-ethyl)amino]-3-methyl-4-[(4-nitrophenyl)azo]-benzol (Cl11210, Disperse Red No. 17), 4-[(4-Aminophenyl)azo]-1-[di (2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-dinatriumsalz (Cl15985; Food Yellow No. 3; FD&C Yellow No. 6), 2,4-Dinitro-1-naphthol-7-sulfonsäure-dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäuretrinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)-amino]-2-phenylaminobenzolsulfonsäure-natriumsalz (Cl10385; Acid Orange No. 3), 4-[(2,4-Dihydroxyphenyl)azo]-benzolsulfonsäure-mononatriumsalz (Cl14270; Acid

Orange No. 6), 4-[(2-Hydroxynaphth-1-yl)azo]-benzolsulfonsäurenatriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalinsulfonsäure-dinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanammonium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'dihydroxyspiro[isobenzofuran-1(3H),9'-[9H]xanthen]-3-on-dinatriumsalz (CI45380; Acid Red No. 87), 2',4,5',7-Tetrabrom-4,5,6,7-tetrachlor-3',6'dihydroxyspiro[isobenzofuran-1(3H),9'[9H]xanthen]-3-on-dinatriumsalz (CI45410; Acid Red No. 92), 3',6'-Dihydroxy-4',5'-diiodospiro-[isobenzofuran-1(3H),9'(9H)-xanthen]-3-on-dinatriumsalz (CI45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinon-dinatriumsalz (CI 61570; Acid Green No. 25), Bis[4-(dimethylamino)phenyl]-(3,7-disulfo-2-hydroxynaphth-1-yl)carbeniuminneres Salz, mononatriumsalz (CI44090; Food Green No. 4; Acid Green No. 50), Bis [4-(diethylamino)phenyl](2,4-disulfophenyl)carbenium-inneres salz, Natriumsalz (2:1) (CI42045; Food Blue No. 3; Acid Blue No. 1). Bis[4-(diethylamino)phenyl](5-hydroxy-2,4-disulfophenyl)carbeniuminneres salz, Calciumsalz (2:1) (CI42051; Acid Blue No. 3), 1-Amino-4-(cyclohexylamino)-9,10-anthrachinon-2-sulfonsäurenatriumsalz (C162045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (C173015; Acid Blue No. 74), 9-(2-Carboxyphenyl)-3-[(2-methylphenyl)-amino]-6-[(2-methyl-4-sulfophenyl) amino]xanthylium-inneres Salz, mononatriumsalz (CI45190; Acid Violet No. 9), 1-Hydroxy-4-[(4-methyl-2-sulfophenyl) amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäuredinatriumsalz (CI20470; Acid Black No. 1), 3-Hydroxy-4-[(2-hydroxynaphth-1-yl) azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäure-dinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäuretetranatriumsalz (CI28440; Food Black No. 1) und 3-Hydroxy-4-(3-methyl-5-oxo-1-phenyl-4,5-dihydro-1H-pyrazol-4-ylazo)-naphthalin-1-sulfonsäurenatriumsalz-Chrom-Komplex (Acid Red No. 195).

Obwohl die in dem erfindungsgemäßen Mittel enthaltenen Polymethinfarbstoffe definitionsgemäß anionischen Charakter besitzen, sind einige dieser Farbstoffe, insbesondere die Monomethinoxonole **(1)** und **(2)**, erstaunlicherweise völlig kompatibel zu kationischen ("basischen") Farbstoffen, beispielsweise 9-(Dimethylamino)-benzo[a] phenoxazin-7-iumchlorid (CI51175; Basic Blue No. 6), Di[4-(diethylamino)phenyl][4-(ethylamino)naphthyl]carbeniumchlorid (CI42595; Basic Blue No. 7), 3,7-Di(dimethylamino)phenothiazin-5-ium-chlorid (CI52015; Basic Blue No. 9), Di [4-(dimethylamino)phenyl][4-(phenyl-amino)naphthyl]carbeniumchlorid (CI44045; Basic Blue No. 26), 2-[(4-(Ethyl (2-hydroxyethyl)amino)-phenyl)azo]-6-methoxy-3-methyl-benzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethyl-amino)phenyl][4-(methylamino)phenyl]carbeniumchlorid (CI42535; Basic Violet No. 1), Tris[4(dimethylamino)phenyl]-carbenium-chlorid (CI42555; Basic Violet No. 3), 2-[3,6-(Diethylamino)-dibenzopyranium-9-yl]-benzoesäure-chlorid (CI45170; Basic Violet No. 10), Di(4-aminophenyl)-(4-amino-3-methylphenyl)carbenium-chlorid (CI42510; Basic Violet No. 14), 1,3-Bis[(2,4-diamino-5-methylphenyl)azo]-3-methylbenzol (CI21010; Basic Brown No. 4), 1-[(4-Aminophenyl)azo]-7-(trimethyl-ammonio)-2-naphthol-chlorid (CI12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalinchlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)azo]-pyrazol-5-onchlorid (CI12719; Basic Yellow No. 57) oder Bis[4-(diethylamino)phenyl]-phenylcarbenium-hydrogensulfat(1:1) (CI42040; Basic Green No. 1).

Der Gesamtgehalt an natürlichen oder synthetischen nicht-oxidativen Farbstoffen in dem erfindungsgemäßen Färbemittel beträgt 0,01 bis 15 Gewichtsprozent, insbesondere 0,1 bis 12 Gewichtsprozent.

Zur Erhöhung der Farbintensität können die in kosmetischen Systemen üblichen Carrier zugesetzt werden. Geeignete Verbindungen sind beispielsweise Benzylalkohol, Furfurylalkohol, 2-Hydroxymethylthiophen, Vanillin oder Isovaniltin. Weitere geeignete Carrier werden in der DE-OS 196 18 595 beschrieben, auf die hiermit ausdrücklich Bezug genommen wird.

Das erfindungsgemäße Mittel zur Färbung von Keratinfasern kann beispielsweise in Form einer Lösung, insbesondere als wäßrigalkoholische Lösung, einer Creme, eines Geles oder einer Emulsion. vorliegen. Als Lösungsmittel können neben Wasser beispielsweise niedere aliphatische einwertige oder mehrwertige Alkohole, deren Ester und Ether, oder aber Gemische dieser Lösungsmittel untereinander oder mit Wasser genannt werden. Der maximale Siedepunkt der vorgenannten geeigneten Lösungsmittel beträgt etwa 400°C, wobei ein Siedepunkt von 20°C bis 250°C bevorzugt ist.

Ebenfalls ist es möglich, dieses Mittel mit Hilfe eines Zerstäubers beziehungsweise anderer geeigneter Pumpvorrichtungen oder Sprühvorrichtungen oder im Gemisch mit üblichen unter Druck verflüssigten Treibmitteln als Aerosolspray oder als Aerosolschaum aus einem Druckbehälter zu entnehmen.

Der pH-Wert des erfindungsgemäßen Färbemittels beträgt 2 bis 11, wobei ein pH-Wert von 2,5 bis 8 besonders bevorzugt ist. Die Einstellung eines alkalischen pH-Wertes erfolgt vorzugsweise mit Ammoniak, es ist jedoch auch möglich, anstelle von Ammoniak organische Amine, wie zum Beispiel Monoethanolamin oder Triethanolamin zu verwenden. Für die Einstellung eines sauren pH-Wertes kann hingegen eine organische oder anorganische Säure, wie zum Beispiel Salzsäure, Schwefelsäure, Phosphorsäure, Ascorbinsäure, Glycolsäure oder Milchsäure, verwendet werden.

Selbstverständlich kann das vorstehend beschriebene Färbemittel gegebenenfalls weitere für Färbemittel für Keratinfasem übliche Zusätze, wie zum Beispiel Pflegestoffe, Netzmittel, Verdicker, Weichmacher, Konservierungsstoffe und Parfümöle sowie auch weitere, nachstehend aufgeführte Zusätze enthalten.

Weiterhin können in dem erfindungsgemäßen Färbemittel Netzmittel oder Emulgatoren aus den Klassen der anionischen, amphoteren, nichtionogenen oder zwitterionischen oberflächenaktiven Substanzen wie Fettalkoholsulfate, Alkansulfonate, Alkylbenzolsulfonate, Alkylbetaine, $\alpha$-Olefinsulfonate, oxethylierte Fettalkohole, oxethylierte Nonylphenole, Fettsäurealkanolamine, oxethylierte Fettsäureester, Fettalkoholpolyglycolethersulfate, Alkylpolyglucoside, Verdickungsmittel wie höhere Fettalkohole, Stärke, Alginate, Bentonite, Cellulosederivate, Vaseline, Paraffinöl und Fettsäuren, wasserlösliche polymere Verdickungsmittel wie natürliche Gummiarten, Guargummi, Xanthangummi, Johannisbrotkemmehl, Pektin, Dextran, Agar-Agar, Amylose, Amylopektin, Dextrine, Tone oder vollsynthetische Hydrokolloide wie Polyvinylalkohol, außerdem Pflegestoffe wie Lanolinderivate, Cholesterin, Pantothensäure, wasserlösliche Polymere, Proteinderivate, Provitamine, Vitamine, Pflanzenextrakte, Zucker und Betain, Hilfsstoffe wie Feuchthaltemittel, Elektrolyte, Antioxidantien, Fettamide, Sequestrierungsmittel, filmbildende Agentien und Konservierungsmittel, enthalten sein.

Das vorstehend beschriebene Färbemittel kann weiterhin natürliche oder synthetische Polymere beziehungsweise modifizierte Polymere natürlichen Ursprungs enthalten sein, wodurch gleichzeitig mit der Färbung eine Festigung der Keratinfaser erreicht wird. Solche Mittel werden im allgemeinen als Tönungsfestiger oder Farbfestiger bezeichnet. Von den für diesen Zweck in der Kosmetik bekannten synthetischen Polymeren seien beispielsweise Polyvinylpyrrolidon, Polyvinylacetat, Polyvinylalkohol oder Polyacrylverbindungen wie Polyacrylsäure oder Polymethacrylsäure, Polyacrylnitril, Polyvinylacetate sowie Copolymerisate aus derartigen Verbindungen, wie zum Beispiel Polyvinylpyrrolidon-Vinylacetat, erwähnt; während als natürliche Polymere oder modifizierte natürliche Polymere beispielsweise Chitosan (deacetyliertes Chitin) oder Chitosanderivate, eingesetzt werden können.

Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Menge von etwa 0,1 bis 5 Gewichtsprozent. Die vorgenannten Polymere können in dem erfindungsgemäßen Mittel in der für solche Mittel üblichen Menge, insbesondere in einer Menge von etwa 1 bis 5 Gewichtsprozent, verwendet werden.

Das erfindungsgemäße Mittel zur Färbung von Keratinfasern eignet sich insbesondere zu Färbung von Haaren. Das erfindungsgemäße Färbemittel wird hierzu in üblicher Weise in einer für die Färbung der Haare ausreichende Menge, im allgemeinen etwa 50 bis 150 Gramm, auf das Haar aufgetragen. Nach einer für die Haarfärbung ausreichenden Einwirkungszeit, die üblicherweise etwa 10 bis 45 Minuten bei 20 bis 50 °C, vorzugsweise 15 bis 30 Minuten bei etwa 40°C, beträgt, wird das Haar mit Wasser gespült, gegebenenfalls mit einem Shampoo gewaschen und/oder mit der wäßrigen Lösung einer schwachen organischen Säure, wie zum Beispiel Zitronensäure oder Weinsäure, nachgespült und getrocknet.

Die Anwendung des Färbemittels mit zusätzlicher Festigung erfolgt in bekannter und üblicher Weise durch Befeuchten des Haares mit dem Festiger, Einlegen des Haares zur Frisur und anschließende Trocknung.

Hinsichtlich der färberischen Möglichkeiten bietet das erfindungsgemäße Färbemittel je nach Art und Zusammensetzung der verwendeten Farbstoffe eine breite Palette verschiedener Farbnuancen, die sich von natürlichen Farbtönen bis hin zu hochmodischen, leuchtenden Nuancen erstreckt. Die vorzüglichen Eigenschaften des neuen Färbemittels zeigen sich insbesondere auf durch Licht und Wetter geschädigtem oder auf dauergewellten Haaren. Hierbei zeichnen sich die erhaltenen Färbungen insbesondere auch durch ihre sehr gute Haltbarkeit und Auswaschbeständigkeit aus.

Die Herstellung von Monomethinoxonolfarbstoffen (m = n = 0) kann aus den entsprechenden Heterocyclen

und Trialkyl-orthoestern oder Formamiden als Brückenvorstufen erfolgen. Zahlreiche Verbindungen sind beschrieben bei B. Schied, J. prakt. Chem. **157**, Seite 203 ff. (1941) und S. Hünig, Annalen, **574,** Seite 106 ff. (1951) sowie der dort zitierten Literatur. Zur Herstellung der Trimethinoxonole (m + n = 1) können nach der DE-OS 20 12 050 Tetramethoxypropan, Trimethoxypropen oder Malonaldehyddianil verwendet werden. Pentamethinoxonole (m + n = 2) lassen sich aus N-akzeptorsubstituierten Pyridiniumsalzen unter Ringöffnung des Pyridinrings ("Zincke-Spaltung") oder einem entsprechenden Derivat, wie zum Beispiel Glutaconaldehyddianil, herstellen. Eine komplette Zusammenfassung darüber findet man in Houben-Weyl 7/1, 4. Auflage (1954) auf den Seiten 263 ff. sowie der dort zitierten Literatur.

Die nachstehenden Beispiele sollen den Erfindungsgegenstand näher erläutern, ohne ihn auf die angeführten Beispiele zu beschränken.

**Beispiele**

**Beispiel 1:** Verfahren zur Herstellung von 2-(2-Hydroxyethyl)-4-(5-hydroxy-1-(2-hydroxyethyl)-3-methyl-1H-pyrazol-4-ylmethylen)-5-methyl-2,4-dihydro-pyrazol-3-on **(2)**

17 g 1-(2-Hydroxyethyl)-3-methyl-1H-pyrazol und 7 g Orthoameisensäuretrimethylester werden in 50 ml Pyridin unter Rückfluß erhitzt. Nach 15 Stunden wird abgekühlt und der Niederschlag abfiltriert. Umkristallisieren aus 220 ml Ethanol ergibt 9,5 g 2-(2-Hydroxyethyl)-4-(5-hydroxy-1-(2-hydroxyethyl)-3-methyl-1H-pyrazol-4-ylmethylen)-5-methyl-2,4-dihydropyrazol-3-on **(2)** in Form vonleuchtend gelbe Nadeln.

| | |
|---|---|
| Schmelzpunkt: | 212-214°C |
| $\lambda_{max}$ ($H_2O$): | 404 nm |
| ε: | 55200 |

| Elementaranalyse: | | C | H | N |
|---|---|---|---|---|
| ($C_{13}H_{18}N_4O_4$) | ber. | 53,05% | 6,16% | 19,04% |
| | gef. | 53,04% | 6,15% | 19,11% |

**Beispiel 2:** Verfahren zur Herstellung von Ammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24b)**

(a) 26,5 g 5-(3-(5-Cyano-1-ethyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitril und 5,2 g Ammoniumacetat werden in 250 ml Essigsäure 2 Stunden lang auf 80°C erhitzt. Man erhält eine dicke Farbstoffsuspension. Anschließend wird die Farbstoffsuspension abgekühlt, der Farbstoff abfiltriert und getrocknet. Es werden 25,5 g Ammonium-5-(3-(5-cyano-1-ethyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitril **(24b)** erhalten.

| | |
|---|---|
| Schmelzpunkt: | 300-301 °C |
| $\lambda_{max}$ (Methanol) : | 596 nm |
| ε: | 177500 |

| Elementaranalyse: | | C | H | N |
|---|---|---|---|---|
| $C_{21}H_{20}N_4O_4$ x $NH_3$ | ber. | 61,60% | 5,66% | 17,11% |
| | gef. | 61,53% | 5,79% | 17,07% |

(b) 4,9 g im Handel erhältliches Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitrit (24a) werden wie unter **(a)** beschrieben mit 0,8 g Ammoniumacetat in 75 ml Essigsäure umgesetzt. Es werden 3,6 g des Farbstoffes **(24b)** erhalten.

**Beispiel 3:** Färbemittel

| | |
|---|---|
| 0,7 g | 2-(2-Hydroxyethyl)-4-(5-hydroxy-1-(2-hydroxyethyl)-3-methyl-1 H-pyrazol-4-ylmethylen)-5-methyl-2,4-dihydropyrazol-3-on **(2)** |
| 0,6 g | 1-[(2-Hydroxyethyl)amino]-4-methylamino-9,10-anthrachinon (CI61505, Disperse Blue No. 3) |
| 20,0 g | Ethanol |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist blaugrün gefärbt.
Nach dem Waschversuch ist visuell ein geringer Farbverlust,
insbesondere zu Lasten des eingesetzten konventionellen Direktziehers (Disperse Blue No. 3) festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 36,30 | -12,60 | -8,04 | 53,63 | |
| 5x gewaschenes und getrocknetes Haar | 42,28 | -15,09 | -5,56 | 6,94 | 13 |

**Beispiel 4:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,7 g | Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)** |
| 1,5 g | 6-Hydroxy-5-[(4-sulfonaphth-1-yl)azo]-2,4-naphthalindisulfonsäure-trinatriumsalz (CI16255; Acid Red No. 18), |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist blauviolett gefärbt.
Nach dem Waschversuch ist visuell kein Farbverlust festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 20,48 | 18,23 | -25,38 | 76,55 | |
| 5x gewaschenes und getrocknetes Haar | 22,71 | 21,44 | -27,47 | 4,43 | 6 |

**Beispiel 5:** Färbemittel

| | |
|---|---|
| 5,0 g | Ethanol |
| 1,5 g | Glycolsäure |
| 2,0 g | Natrium-cocoamphoacetat (50%ige wäßrige Lösung) |

(fortgesetzt)

| | |
|---|---|
| 5,0 g | Benzylalkohol |
| 1,4 g | Dinatrium-4-(3-(5-hydroxy-3-methyl-1-(4-sulfophenyl)-1H-pyrazol-4-yl)-allyliden)-5-methyl-2-(4-sulfophenyl)-2,4-dihydro-pyrazol-3-on **(7a)** |
| 0,5 g | 4-[(2-Hydroxyethyl)-amino]-3-nitro-1-methylbenzol |
| ad 100,0 g | Wasser, demineralisiert |

Die obige Färbelösung, wird auf gebleichtes Haar aufgetragen. Nach einer Einwirkungszeit von 20 Minuten bei 40°C wird mit Wasser ausgewaschen und getrocknet. Das Haar ist leuchtend rot gefärbt.
Nach dem Waschversuch ist visuell kein Farbverlust festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 39,39 | 65,56 | 36,61 | 84,19 | |
| 5x gewaschenes und getrocknetes Haar | 39,51 | 66,38 | 37,32 | 1,09 | 1 |

**Beispiel 6:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,8 g | Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)** |
| 0,1 g | 5-Chlor-1,4-[di(2,3-dihydroxypropyl)amino] -2-nitrobenzol (HC Red No. 11) |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist in einem dunklen Auberginеton gefärbt. Nach dem Waschversuch ist visuell ein schwacher Farbverlust festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 18,43 | 2,83 | -2,39 | 67,91 | |
| 5x gewaschenes und getrocknetes Haar | 21,35 | 3,37 | -3,87 | 3,32 | 5 |

**Beispiel 7:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 0,7 g | 2-(2-Hydroxyethyl)-4-(5-hydroxy-1-(2-hydroxyethyl)-3-methyl-1 H-pyrazol-4-ylmethylen)-5-methyl-2,4-dihydropyrazol-3-on **(2)** |

(fortgesetzt)

| | |
|---|---|
| 0,6 g | Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1 H-pyrazol-3-carbonsäure **(15a)** |
| 0,5 g | 2-Amino-6-chlor-4-nitrophenol-Hydrochlorid |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist in einem mittleren Braunton gefärbt. Nach dem Waschversuch ist visuell kein Farbverlust festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 25,14 | 2,16 | 11,89 | 59,73 | |
| 5x gewaschenes und getrocknetes Haar | 24,07 | 3,21 | 10,42 | 2,10 | 4 |

**Beispiel 8:** Färbemittel

| | |
|---|---|
| 5,0 g | Ethanol |
| 1,5 g | Glycolsäure |
| 2,0 g | Natrium-cocoamphoacetat (50%ige wäßrige Lösung) |
| 5,0 g | Benzylalkohol |
| 1,2 g | Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril **(24a)** |
| 0,8 g | Tetranatrium-4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure **(9a)** |
| 0,5 g | Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure **(15a)** |
| 0,7 g | 5-Hydroxy-1-(4-sulfophenyl)-4-[(4-sulfophenyl)-azo]-pyrazol-3-carbonsäure-trinatriumsalz (CI19140; Acid Yellow No. 23), |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist in einem grünstichigen Blauton gefärbt. Nach dem Waschversuch ist visuell kaum ein Farbverlust festzustellen.

| | L* | a* | b* | $\Delta E_{1/2}$ | Farbverlust [%] |
|---|---|---|---|---|---|
| unbehandeltes Haar | 84,80 | -0,75 | 11,53 | | |
| gefärbtes Haar | 21,94 | 5,95 | -11,69 | 67,35 | |
| 5x gewaschenes und getrocknetes Haar | 24,63 | 4,68 | -11,94 | 2,99 | 4 |

**Beispiel 9:** Färbemittel

| | |
|---|---|
| 10,0 g | Ethanol |
| 10,0 g | Natriumlaurylalkohol-diglycolethersulfat (28%ige wäßrige Lösung) |
| 10,0 g | Ammoniak (25%ige wäßrige Lösung) |

(fortgesetzt)

| | |
|---|---|
| 0,7 g | 2-(2-Hydroxyethyl)-4-(5-hydroxy-1-(2-hydroxyethyl)-3-methyl-1 H-pyrazol-4-ylmethylen)-5-methyl-2,4-dihydropyrazol-3-on **(2),** |
| 1,0 g | Ammonium5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24b)** |
| 1,1 g | 2-Chlor-6-ethylamino-4-nitrophenol |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist mittelbraun gefärbt.

**Beispiel 10:** Färbemittel (Creme sauer)

| | |
|---|---|
| 2,0 g | Natrium-laurylsulfat |
| 20,0 g | Cetearylalkohol |
| 5,0 g | Glycerylstearat SE |
| 2,0 g | Lanolin Alkohol |
| 12.5 g | Laurylethersulfat (28%ige wäßrige Lösung) |
| 0,3 g | Ammonium5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24b)** |
| 0,4 g | 2-Chlor-6-ethylamino-4-nitrophenol |
| 0,2 g | N-(2,3-Dihydroxypropyl)-2-nitro-4-(trifluormethyl)-anilin |
| ad 100,0 g | Wasser, demineralisiert. |

Die Creme wird vor Gebrauch 1:1 mit Wasser angemischt und auf gebleichtes Haar aufgetragen. Nach einer Einwirkungszeit von 20 Minuten bei 40°C wird mit einem Shampoo ausgewaschen und getrocknet.
Das Haar ist mittelblond gefärbt.

**Beispiel 11:** Färbemittel (Kombination mit kationischem Direktzieher)

| | |
|---|---|
| 10,0 g | Ethanol |
| 10,0 g | Polyethylenlaurylether (25%ige wäßrige Lösung) |
| 0,5 g | 2-(2-Hydroxyethyl)-4-(5-hydroxy-1-(2-hydroxyethyl)-3-methyl-1 H-pyrazol-4-ylmethylen)-5-methyl-2,4-dihydropyrazol-3-on (2), |
| 0,2 g | Di[4-(diethylamino)phenyl][4-(ethylamino)naphthyl]carbenium-chlorid (Cl42595; Basic Blue No. 7), |
| ad 100,0 g | Wasser, demineralisiert |

Die obige Färbelösung wird auf gebleichtes Haar aufgetragen. Nach einer Einwirkungszeit von 20 Minuten bei 40°C wird mit Wasser ausgewaschen und getrocknet. Das Haar ist dunkelgrün gefärbt.

**Beispiel 12:** Färbemittel mit Carrier

| | |
|---|---|
| 5,0 g | Ethanol |
| 2,0 g | Milchsäure |
| 2,0 g | Natrium-cocoamphoacetat (50prozentige wäßrige Lösung) |
| 5,0 g | Carrier gemäß nachfolgender Tabelle 1 |
| 1,2 g | Ammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril **(24b)** |

(fortgesetzt)

| | |
|---|---|
| 0,4 g | 4-Amino-3-nitrophenol |
| ad 100,0 g | Wasser, demineralisiert |

Die Färbelösung wird auf gebleichtes Haar aufgetragen. Nach 20 Minuten Einwirkzeit bei 40 °C wird mit Wasser ausgewaschen und getrocknet. Das Mittel ohne Carrier ergibt einem mittleren Blauton, die Mittel mit Carrier führen zu intensiven Blautönen.

Tabelle 1

| Carrier | Farbergebnis | Anmerkung |
|---|---|---|
| **ohne Carrier** | rotbraun | - |
| 5x waschen und trocknen | stark blaustichiger Braunton | Gelbkomponente wäscht sich aus |
| **2-Hydroxy-methylthiophen** | gesättigtes Rotbraun | - |
| 5x waschen und trocknen | gesättigtes Rotbraun | kein meßbarer Unterschied der Färbung, unempfindlich gegen Shampoonieren |

Die in den vorliegenden Beispielen angegebenen L*a*b*-Farbmesswerte wurden mit einem Farbmessgerät der Firma Minolta, Typ Chromameter II, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

Der Wert ΔE gibt die Farbdifferenz an, die zwischen dem unbehandelten und dem gefärbten Haar beziehungsweise dem gefärbten und dem gewaschenen Haar besteht. Er wird folgendermaßen bestimmt:

$$\Delta E = \sqrt{(L_i - L_0)^2 + (a_i - a_0)^2 + (b_i - b_0)^2}$$

wobei $L_0$, $a_0$ und $b_0$ die Farbmesswerte vor der Färbung beziehungsweise dem Waschversuch bedeuten und $L_i$, $a_i$ und $b_i$ die Werte nach der Färbung beziehungsweise Waschversuch bedeuten.

Der Auswaschgrad in Prozent wurde nach der folgenden Formel ermittelt:

$$\text{Entfärbegrad [\%]} = [1 - (\Delta E_2 / \Delta E_1)] \times 100$$

Hierbei steht $\Delta E_1$ für den Färbeschritt und $\Delta E_2$ für den Auswaschschritt.

Alle in der vorliegenden Anmeldung genannten Prozentangaben stellen soweit nicht anders angegeben Gewichtsprozente dar.

## Patentansprüche

1. Mittel zur Färbung von Keratinfasern, **dadurch gekennzeichnet, daß** es eine Kombination aus (a) mindestens einem natürlichen oder synthetischen nicht-oxidativen Farbstoff, welcher ausgewählt ist aus natürlichen Farbstoffen oder Nitrofarbstoffen, Azofarbstoffen, Chinonfarbstoffen, Triphenylmethanfarbstoffen und basischen oder sauren Farbstoffen, und (b) mindestens einem Polymethinfarbstoff der allgemeinen Formeln (I) bis (III) oder deren physiologisch verträglichen Salze, enthält,

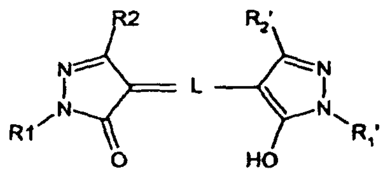

(I)

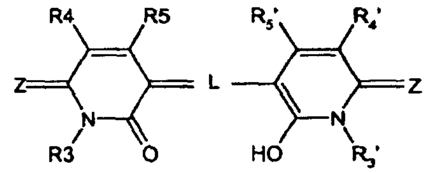

(II)

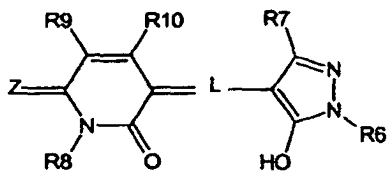

(III)

worin **R1, R1' und R6** Wasserstoff, eine geradkettige oder verzweigte C1- bis C8- Alkylgruppe, eine Hydroxyethylgruppe, eine Dihydroxypropylgruppe, eine Methoxyethylgruppe, eine Carboxyethylgruppe, eine C1- bis C4-Sulfoalkylgruppe, einen Phenylrest oder einen mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierten Phenylrest, einen Benzylrest oder einen mit einem oder mehreren Halogenatomen, mit einer C1- bis C4-Alkylgruppe, einer Hydroxygruppe, einer Methoxygruppe, einer Carboxygruppe, einer Nitrogruppe oder einer Aminogruppe substituierten Benzylrest oder einen fünfgliedrigen oder sechsgliedrigen gesättigten oder ungesättigten Heterocyclus bedeuten, wobei **R1 und R1'** gleich oder verschieden sein können, und

**R2, R2' und R7** Wasserstoff, eine verzweigte oder unverzweigte C1- bis C6- Alkylgruppe, einen Phenylrest, eine Aminogruppe, die zusätzlich acyliert oder sulfonyliert sein kann, eine Acetylgruppe, eine Methoxygruppe, eine Carbonsäuregruppe, die mit einem geradkettigen oder verzweigten C1- bis C8-Alkohol oder einem Ethylenglykolmonomethylether oder einem Ethylenglykolmonoethylether verestert sein kann, eine Carbonsäureamidgruppe, eine Carbonsäureanilidgruppe, eine 2-Amino-2-oxyethylgruppe oder eine Nitrilgruppe bedeuten, wobei R2 und R2' gleich oder verschieden sein können, und

**R3, R3' und R8** Wasserstoff, eine geradkettige oder verzweigte C1- bis C11-Alkylgruppe, eine geradkettige oder verzweigte C1- bis C11-Monohydroxyalkylgruppe, eine geradkettige oder verzweigte C1- bis C11-Dihydroxyalkylgruppe, eine geradkettige oder verzweigte C1- bis C11-Alkoxyalkylgruppe, eine geradkettige oder verzweigte C1- bis C11-Monoalkylaminogruppe, eine Aminogruppe der Formel $(CH_2)_x$-NR11R12 (mit x gleich einer ganzen Zahl von 0 bis 3 und R11 und R12 unabhängig voneinander gleich einer C1- bis C3-Alkylgruppe), eine C2- bis C4-Sulfoalkylgruppe oder Carboxyalkylgruppe, eine Phenylgruppe oder eine mit einem oder mehreren Halogenatomen, einer oder zwei Sulfonsäuregruppen, einer oder zwei Carbonsäuregruppen, einer oder mehreren unverzweigten oder verzweigten C1- bis C8-Alkylgruppen oder C1- bis C8-Alkoxygruppen substituierte Phenylgruppe, eine Benzylgruppe oder eine mit einem oder mehreren Halogenatomen, mit einer C1- bis C4-Alkylgruppe, einer Hydroxygruppe, einer Methoxygruppe, einer Nitrogruppe oder einer Aminogruppe substituierte Benzylgruppe, eine Phenylethylgruppe, ein fünfgtiedriger oder sechsgliedriger aromatischer oder nicht-aromatischer Heterocyclus, der direkt oder über eine Methylengruppe gebunden sein kann, eine Pyrrolidinogruppe , Morpholinogruppe , Piperazinogruppe , Piperidinogruppe oder Pyridino(C2- oder C3-)atkylgruppe oder eine Trialkylammoniumalkylgruppe der Formel $R13\text{-}N(R14)_3^+$ (mit R13 gleich einer C1- bis C6-Alkylengruppe und R14 gleich einer Methyl- oder Ethylgruppe, wobei die Gesamtzahl an Kohlenstoffatomen im Molekül gleich 5 bis 9 ist) bedeuten, wobei **R3 und R3'** gleich oder verschieden sein können, und

**R4, R4' und R9** Wasserstoff, eine Nitrilgruppe. eine Carbonsäureestergruppe, eine Carbonsäureamidgruppe, eine Sulfonsäuregruppe, eine Sulfomethylgruppe, eine Methansulfonylgruppe, einen Pyridiniumrest oder einen Imidazoliumrest bedeuten, wobei **R4 und R4'** gleich oder verschieden sein können, und

**R5, R5' und R10** Wasserstoff, eine C1- bis C4-Alkylgruppe, eine C5- bis C6-Cyctoalkytgnrppe, eine Phenytgruppe, eine Methoxyphenylgruppe, eine Benzylgruppe, eine Phenylethylgruppe oder eine Carbonsäuregruppe bedeuten, wobei **R5 und R5'** gleich oder verschieden sein können, und

**Z** für Sauerstoff oder einen Rest der Formel $C(CN)_2$, C(CN)COOQ oder $C(COOQ)_2$, worin Q jeweils eine C1- bis C8-Alkylgruppe oder einen Ethylenglykolmono(C3- bis C7-)alkytether darstellt, steht, und
**L** für eine Brückengruppe der allgemeinen Formel

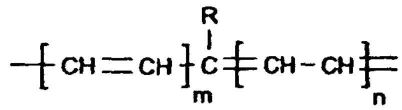

steht, worin **R** ein Wasserstoffatom, eine Phenylgruppe, eine Methylgruppe, eine Carbonamidgruppe oder ein Halogenatom darstellt, und die Indices **m** und **n** jeweils gleich 0, 1 oder 2 sein können, wobei die Summe aus m und n nicht größer als 2 ist.

2. Mittel nach Anspruch 1, **dadurch gekennzeichnet, daß** das physiologisch verträgliche Salz ausgewählt ist aus Ammoniumsalzen, Natriumsalzen, Kaliumsalzen, Triethylammoniumsalzen, N-Methylmorpholiniumsalzen, Monoethanolammoniumsalzen, Diethanolammoniumsalzen und Triethanolammoniumsalzen.

3. Mittel nach Anspruch 1, **dadurch gekennzeichnet, daß** die Farbstoffe der Formel (I) bis (III) ausgewählt sind aus 4-(5-Hydroxy-1,3-dimethyl-1 H-pyrazol-4-ylmethylen)-2,5-dimethyl-2,4-dihydropyrazol-3-on; 2-(2-Hydroxyethyl)-4-(5-hydroxy-1-(2-hydroxyethyl)-3-methyl-1H-pyrazol-4-ylmethylen)-5-methyl-2,4-dihydropyrazol-3-on; 4-(5-Hydroxy-3-methyl-1-(4-sulfophenyl)-1H-pyrazol-4-ylmethylen)-5-methyl-2-(4-sulfophenyl)-2,4-dihydropyrazol-3-on; Diammonium-4-(5-hydroxy-3-methyl-1-(4-sulfophenyl)-1H-pyrazol-4-yl-methylen)-5-methyl-2-(4-sulfophenyl)-2,4-dihydro-pyrazol-3-on; 4-(3-(5-Hydroxy-1,3-dimethyl-1H-pyrazol-4-yl)-allyliden)-2,5-dimethyl-2,4-dihydropyrazol-3-on; 5-Amino-4-(3-(3-amino-5-hydroxy-1-phenyl-1 H-pyrazol-4-yl)-allyliden)-2-phenyl-2,4-dihydropyrazol-3-on; 2-(2-Hydroxyethyl)-4-(3-(5-hydroxy-1-(2-hydroxyethyl)-3-methyl-1H-pyrazol-4-yl)-allyliden)-5-methyl-2,4-dihydropyrazol-3-on; 4-(3-(5-Hydroxy-3-methyl-1-(4-sulfophenyl)-1H-pyrazol-4-yl)-allyliden)-5-methyl-2-(4-sulfophenyl)-2,4-dihydro-pyrazol-3-on; Dinatrium-4-(3-(5-hydroxy-3-methy)-1-(4-sulfophenyl)-1H-pyrazol-4-yl)-allyliden)-5-methyl-2-(4-sulfophenyl)-2,4-dihydro-pyrazol-3-on; 4-(3-(5-Hydroxy-3-methyl-1-(4-sulfophenyl)-1H-pyrazol-4-yl)-but-2-enyliden)-5-methyl-2-(4-sulfophenyl)-2,4-dihydropyrazol-3-on; Trikalium-4-(3-(5-hydroxy-3-methyl-1-(4-sulfophenyl)-1H-pyrazol-4-yl)-but-2-enyliden)-5-methyl-2-(4-sulfophenyl)-2,4-dihydro-pyrazol-3-on; 4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure; Tetranatrium-4-(3-(3-Carboxy-5-oxo-1-(4-sulfophenyl)-1,5-dihydropyrazol-4-yliden)-propenyl)-5-hydroxy-1-(4-sulfophenyl)-1H-pyrazol-3-carbonsäure; 5-Hydroxy-4-(3-(3-methoxycarbonyl-5-oxo-1-(4-sulfophenyl)-1,5-dihydropyrazol-4-yliden)-propenyl)-1-(4-sulfophenyl)-1H-pyrazol-3-carbonsäuremethylester; 4-(5-(5-Hydroxy-1,3-dimethyl-1H-pyrazol-4-yl)-penta-2,4-dienyliden)-2,5-dimethyl-2,4-dihydropyrazol-3-on; 5-Amino-4-(5-(3-amino-5-hydroxy-1-phenyl-1Hpyrazol-4-yl)-penta-2,4-dienyliden)-2-phenyl-2,4-dihydropyrazol-3-on; 2-(2-Hydroxyethyl)-4-(5-(5-hydroxy-1-(2-hydroxyethyl)-3-methyl-1Hpyrazol-4-yl)-penta-2,4-dienyliden)-5-methyl-2,4-dihydro-pyrazol-3-on; 4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on; Dikalium-4-(5-(5-Hydroxy-3-methyl-1-(4-sulfophenyl)-1H-pyrazol-4-yl)-penta-2,4-dienyliden)-5-methyl-2-(4-sulfophenyl)-2,4-dihydropyrazol-3-on; 4-(5-(3-Carboxy-5-oxo-1-(4-sulfophenyl)-1,5-dihydropyrazol-4-yliden)-penta-1,3-dienyl)-5-hydroxy-1-(4-sulfophenyl)-1H-pyrazol-3-carbonsäure; Dikalium-4-(5-(3-carboxy-5-oxo-1-(4-sulfo-phenyl)-1,5-dihydro-pyrazol-4-yliden)-penta-1,3-dienyl)-5-hydroxy-1-(4-sulfo-phenyl)-1H-pyrazol-3-carbonsäure; 6-Hydroxy-1-(2-hydroxyethyl)-5-(3-(1-(2-hydroxyethyl)-3-methyl-5-oxo-1,5-dihydro-pyrazol-4-yliden)-propenyl)-4-methyl-2-oxo-1,2-dihydro-pyridin-3-carbonitril; 6-Hydroxy-5-(3-(1-(2-hydroxyethyl)-3-methyl-5-oxo-1,5-dihydropyrazol-4-yliden)-propenyl)-4-methyl-2-oxo-1-(4-sulfophenyl)-1,2-dihydropyridin-3-carbonitril; 6-Hydroxy-4-methyl-5-(3-(3-methyl-5-oxo-1-(4-sulfophenyl)-1,5-dihydropyrazol-4-yliden)-propenyl)-2-oxo-1-(4-sulfophenyl)-1,2-dihydropyridin-3-carbonitril; 6-Hydroxy-4-methyl-5-(3-(3-methyl-5-oxo-1-phenyl-1,5-dihydropyrazol-4-yliden)-propenyl)-2-oxo-1-(3-sulfophenyl)-1,2-dihydropyridin-3-carbonitril; 4-(3-(5-Cyano-2-hydroxy-4-methyl-6-oxo-1-phenyl-1,6-dihydropyridin-3-yl)-allyliden)-5-oxo-1-(4-sulfophenyl)-4,5-dihydro-1H-pyrazol-3-carbonsäure; 6-Hydroxy-5-(3-(1-(2-hydroxyethyl)-3-methyl-5-oxo-1,5-dihydropyrazol-4-yliden)-propenyl)-4-methyl-2-oxo-1-phenyl-1,2-dihydropyridin-3-carbonitril; 5-(3-(4-Carboxy-2,6-dioxo-1,6-dihydro-2H-pyridin-3-yliden)-propenyl)-6-hydroxy-2-oxo-1,2-dihydropyridin-4-carbonsäure; 2-(3-Cyano-5-(3-(5-cyano-6-dicyanomethylen-4-methyl-2-oxo-1,6-dihydro-2H-pyridin-3-yliden)-propenyl)-6-hydroxy-4-methyl-1H-pyridin-2-yliden)-malononitril; 5-(3-(5-Cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonitril; Triethylammonium-5-(3-(5-cyano-1-ethyl-4-methyl-2,6-dioxo-1,6-dihydro-2H-pyridin-3-yliden)-propenyl)-1-ethyl-6-hydroxy-4-methyl-2-oxo-1,2-dihydro-pyridin-3-carbonitril; 5-(3-(5-Cyano-1-(2-hydroxyethyl)-4-methyl-2,6-dioxo-1,6-dihydro-2H-pyridin-3-yliden)-propenyl)-6-hydroxy-1-(2-hydroxyethyl)-4-methyl-2-oxo-1,2-dihydropyridin-3-carbonitril; 5-(3-(5-Cyano-1-ethyl-4-(4-methoxyphenyl)-2,6-dioxo-1,6-dihydro-2Hpyridin-3-yliden)-propenyl)-1-ethyl-6-hydroxy-4-(4-methoxyphenyl)-2-oxo-1,2-dihydropyridin-3-carbonitril; 1-Butyl-5-(3-(1-butyl-5-cyano-4-methyl-2,6-dioxo-1,6-dihydro-2H-pyridin-3-yliden)-propenyl)-6-hydroxy-4-methyl-2-oxo-1,2-dihydropyridin-3-carbonitril; 5-(3-(5-Cyano-1-(3-methoxypropyl)-4-methyl-2,6-dioxo-1,6-dihydro-2H-pyridin-3-yliden)-propenyl)-6-hydroxy-1-(3-methoxypropyl)-4-methyl-2-oxo-1,2-dihydropyridin-3-carbonitril; 5-(3-(5-Cyano-1-cyclohexyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitril; 5-(3-(5-Cyano-4-methyl-1-(2-morpholin-4-ylethyl)-2,6-dioxo-1,6-dihydro-2H-pyridin-3-yliden)-propenyl)-6-hydroxy-4-methyl-1-(2-morpholin-4-yl-ethyl)-2-oxo-1,2-dihydropyridin-3-carbonitril;5-(3-(5-Cyano-4-methyl-2,6-dioxo-1-(4-sulfophenyl)-1,6-dihydro-2Hpyridin-3-yliden)-propenyl)-6-hydroxy-4-methyl-2-oxo-1-(4-sulfophenyl)-1,2-dihydropyridin-3-carbonitril; 5-(3-(5-Cyano-4-methyl-2,6-dioxo-1-(3-sulfophenyl)-1,6-dihydro-2H-pyridin-3-yliden)-propenyl)-6-hydroxy-4-methyl-2-oxo-1-(3-sulfophenyl)-1,2-dihydropyridin-3-carbonitril; 5-(3-(5-Cyano-1-dimethylamino-4-methyl-2,6-dioxo-1,6-dihydro-2Hpyridin-3-yliden)-propenyl)-1-dimethylamino-6-hydroxy-4-methyl-2-oxo-1,2-dihydropyridin-3-carbonitril; 5-(3-(5-Carbamoyl-1-ethyl-4-methyl-2,6-dioxo-1,6-dihydro-2H-pyridin-3-yliden)-propenyl)-1-ethyl-6-hydroxy-4-methyl-2-oxo-1,2-dihydropyridin-3-carbonsäureamid; 1-Ethyl-3-(3-(1-ethyl-2-hydroxy-5-methansulfonyl-4-methyl-6-oxo-1,6-dihydro-pyridin-3-yl)-allyliden)-5-methansulfonyl-4-methyl-3H-pyridin-2,6-dion; 5-(5-(5-Cyano-1-ethyl-4-methyl-2,6-dioxo-1,6-dihydro-2H-pyridin-3-yliden)-penta-1,3-dienyl)-1-ethyl-6-hydroxy-4-methyl-2-oxo-1,2-dihydropyridin-3-carbonitril und Ammonium-5-(3-(5-cyano-1-ethyl-4-methyl-2,6-dioxo-1,6-dihydro-2Hpyridin-3-yliden)-propenyl)-1-ethyl-6-hydroxy-4-methyl-2-oxo-1,2-dihydropyridin-3-carbonitrit.

4. Mittel nach einem der Ansprüche 1 bis 3, **dadurch gekennzeichnet, daß** es 0,01 bis 5 Gewichtsprozent der Farbstoffe der Formeln (I) bis (III) enthält.

5. Mittel nach einem der Ansprüche 1 bis 4, **dadurch gekennzeichnet, daß** es 0,01 bis 15 Gewichtsprozent mindestens eines nicht-oxidativen Farbstoffes enthält.

6. Mittel nach einem der Ansprüche 1 bis 5, **dadurch gekennzeichnet, daß** es einen pH-Wert von 2 bis 11 aufweist.

7. Mittel nach einem der Ansprüche 1 bis 6, **dadurch gekennzeichnet, daß** es ein Haarfärbemittel ist.

8. Verfahren zum semipermanenten Färben von Haaren, **dadurch gekennzeichnet, daß** 50 bis 150 Gramm eines Mittels nach einem der Ansprüche 1 bis 7 auf das Haar aufgetragen werden und das Haar nach einer Einwirkungszeit von 10 bis 45 Minuten bei 20 bis 50 °C mit Wasser gespült wird, gegebenenfalls mit einem Shampoo gewaschen und/oder mit der wäßrigen Lösung einer schwachen organischen Säure wie zum Beispiel Zitronensäure oder Weinsäure nachgespült und sodann getrocknet wird.

## Claims

1. Agent for the colouring of keratin fibres, **characterized in that** it comprises a combination of (a) at least one natural or synthetic nonoxidative dye which is chosen from natural dyes or nitro dyes, azo dyes, quinone dyes, triphenylmethane dyes and basic or acidic dyes, and (b) at least one polymethine dye of the general formulae (I) to (III) or physiologically compatible salts thereof,

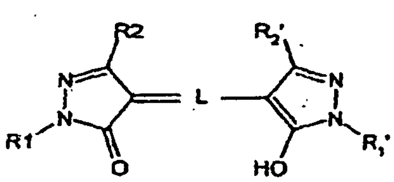

(I)

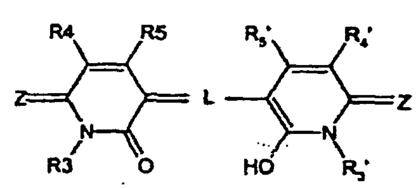

(II)

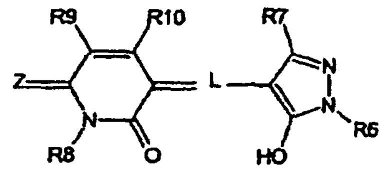

(III)

in which **R1, R1' and R6** are hydrogen, a straight-chain or branched C1- to C8-alkyl group, a hydroxyethyl group, a dihydroxypropyl group, a methoxyethyl group, a carboxyethyl group, a C1- to C4-sulphoalkyl group, a phenyl radical or a phenyl radical substituted by one or more halogen atoms, one or more sulphonic acid groups, one or more carboxylic acid groups, one or more unbranched or branched C1- to C8-alkyl groups or C1- to C8-alkoxy groups, a benzyl radical or a benzyl radical substituted by one or more halogen atoms, by a C1- to C4-alkyl, a hydroxyl group, a methoxy group, a carboxy group, a nitro group or an amino group, or a five-membered or six-membered saturated or unsaturated heterocycle, where **R1 and R1'** may be identical or different, and
**R2, R2' and R7** are hydrogen, a branched or unbranched C1- to C6-alkyl group, a phenyl radical, an amino group, which may additionally be acylated or sulphonylated, an acetyl group, a methoxy group, a carboxylic acid group, which may be esterified by a straight-chain or branched C1-to C8-alcohol or an ethylene glycol monomethyl ether or an ethylene glycol monoethyl ether, a carboxamide group, a carboxanilide group, a 2-amino-2-oxyethyl group or a nitrile group, where **R2 and R2'** may be identical or different, and
**R3, R3' and R8** are hydrogen, a straight-chain or branched C1- to C11-alkyl group, a straight-chain or branched C1- to C11-monohydroxyalkyl group, a straight-chain or branched C1- to C11-dihydroxyalkyl group, a straight-chain or branched C1- to C11-alkoxyalkyl group, a straight-chain or branched C1- to C11-monoalkylamino group, an amino group of the formula $(CH_2)_x$-NR11R12 (where x is an integer from 0 to 3 and R11 and R12, independently of one another, are a C1- to C3-alkyl group), a C2- to C4-sulphalkyl group or carboxyalkyl group, a phenyl group or a phenyl group substituted by one or more halogen atoms, one or more sulphonic acid groups, one or more carboxylic acid groups, one or more unbranched or branched C1- to C8-alkyl groups or C1- to C8-alkoxy groups, a benzyl group or a benzyl group substituted by one or more halogen atoms, by a C1- to C4-alkyl group, a hydroxyalkyl group, a methoxy group, a nitro group or an amino group, a phenylethyl group, a five-membered or six-membered aromatic or nonaromatic heterocycle, which may be bonded directly or via a methylene group, a pyrrolidino group, morpholino group, piperazino group, piperidino group or pyridine(C2- or C3-)alkyl group or a trialkylammonium alkyl group of the formula $R13\text{-}N(R14)_3^+$ (where R13 is a C1- to C6-alkylene group and R14 is a methyl or ethyl group, where the total number of carbon atoms in the molecule is 5 to 9), where **R3 and R3'** may be identical or different, and **R4, R4' and R9** are hydrogen, a nitrile group, a carboxylic ester group, a carboxamide group, a sulphonic acid group, a sulphomethyl group, a methanesulphonyl group, a pyridinium radical or an imidazolium radical, where **R4 and R4'** may be identical or different, and
**R5, R5' and R10** are hydrogen, a C1- to C4-alkyl group, a C5- to C6-cycloalkyl group, a phenyl group, a methoxyphenyl group, a benzyl group, a phenylethyl group or a carboxylic acid group, where **R5 and R5'** may be identical or different, and **Z** is oxygen or a radical of the formula $C(CN)_2$, C(CN)COOQ or $C(COOQ)_2$, in which Q is in each case a C1- to C8-alkyl group or an ethylene glycol mono(C3- to C7-)alkyl ether, and
**L** is a bridging group of the general formula

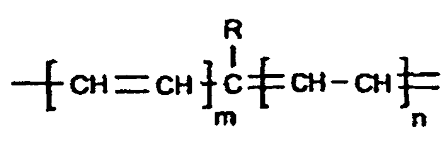

in which **R** is a hydrogen atom, a phenyl group, a methyl group, a carboxamide group or a halogen atom, and the indices **m** and **n** may in each case be 0, 1 or 2, where the sum of m and n is not greater than 2.

**2.** Agent according to Claim 1, **characterized in that** the physiologically compatible salt.is chosen from ammonium salts, sodium salts, potassium salts, triethylammonium salts, N-methylmorpholinium salts, monoethanolammonium salts, diethanolammonium salts and triethanolammonium salts.

3. Agent according to Claim 1, **characterized in that** the dyes of the formula (I) to (III) are chosen from 4-(5-hydroxy-1,3-dimethyl-1H-pyrazol-4-yl-methylene)-2,5-dimethyl-2,4-dihydropyrazol-3-one; 2-(2-hydroxyethyl-4-(5-hydroxy-1-(2-hydroxyethyl)-3-methyl-1H-pyrazol-4-ylmethylene)-5-methyl-2,4-dihydropyrazol-3-one; 4-(5-hydroxy-3-methyl-1-(4-sulphophenyl)-1H-pyrazol-4-ylmethylen)-5-methyl-2-(4-sulphophenyl)-2,4-dihydropyrazol-3-one; diammonium 4-(5-hydroxy-3-methyl-1-(4-sulphophenyl)-1H-pyrazol-4-ylmethylene)-5-methyl-2-(4-sulphophenyl)-2,4-dihydropyrazol-3-one; 4-(3-(5-hydroxy-1,3-dimethyl-1H-pyrazol-4-yl)allylidene)-2,5-dimethyl-2,4-dihydropyrazol-3-one; 5-amino-4-(3-(3-amino-5-hydroxy-1-phenyl-1H-pyrazol-4-yl)allylidene)-2-phenyl-2,4-dihydropyrazol-3-one; 2-(2-hydroxyethyl)-4-(3-(5-hydroxy-1-(2-hydroxyethyl)-3-methyl-1H-pyrazol-4-yl)allylidene)-5-methyl-2,4-dihydropyrazol-3-one; 4-(3-(5-hydroxy-3-methyl-1-(4-sulphophenyl)-1H-pyrazol-4-yl)allylidene)-5-methyl-2-(4-sulphophenyl)-2,4-dihydropyrazol-3-one; disodium 4-(3-(5-hydroxy-3-methyl-1-(4-sulphophenyl)-1H-pyrazol-4-yl)allylidene)-5-methyl-2-(4-sulphophenyl)-2,4-dihydropyrazol-3-one; 4-(3-(5-hydroxy-3-methyl-1-(4-sulphophenyl)-1H-pyrazol-4-yl)but-2-enylidene)-5-methyl-2-(4-sulphophenyl-2,4-dihydropyrazol-3-one; tripotassium 4-(3-(5-hydroxy-3-methyl-1-(4-sulphophenyl)-1H-pyrazol-4-yl)but-2-enylidene)-5-methyl-2-(4-sulphophenyl)-2,4-dihydropyrazol-3-one; 4-(3-(3-carboxy-5-oxo-1-(4-sulphophenyl)-1,5-dihydropyrazol-4-ylidene)propenyl)-5-hydroxy-1-(4-sulphophenyl)-1H-pyrazole-3-carboxylic acid; tetrasodium 4-(3-(3-carboxy-5-oxo-1-(4-sulphophenyl)-1,5-dihydropyrazol-4-ylidene)propenyl)-5-hydroxy-2-(4-sulphophenyl)-2H-pyrazole-3-carboxylic acid; methyl 5-hydroxy-4-(3-(3-methoxycarbonyl-5-oxo-1-(4-sulphophenyl)-1,5-dihydropyrazol-4-ylidene)propenyl)-1-(4-sulphophenyl)-1H-pyrazole-3-carboxylate; 4-(5-(5-hydroxy-1,3-dimethyl-1H-pyrazol-4-yl)penta-2,4-dienylidene)-2,5-dimethyl-2,4-dihydropyrazol-3-one; 5-amino-4-(5-(3-amino-5-hydroxy-1-phenyl-1H-pyrazol-4-yl)penta-2,4-dienylidene)-2-phenyl-2,4-dihydropyrazol-3-one; 2-(2-hydroxyethyl)-4-(5-(5-hydroxy-1-(2-hydroxyethyl)-3-methyl-1H-pyrazol-4-yl)penta-2,4-dienylidene)-5-methyl-2,4-dihydropyrazol-3-one; 4-(5-(5-hydroxy-3-methyl-1-(4-sulphophenyl)-1H-pyrazol-4-yl)penta-2,4-dienylidene)-5-methyl-2-(4-sulphophenyl)-2,4-dihydropyrazol-3-one; dipotassium 4-(5-(5-hydroxy-3-methyl-1-(4-sulphophenyl)-1H-pyrazol-4-yl)penta-2,4-dienylidene)-5-methyl-2-(4-sulphophenyl)-2,4-dihydropyrazol-3-one; 4-(5-(3-carboxy-5-oxo-1-(4-sulphophenyl)-1,5-dihydropyrazol-4-ylidene) penta-1,3-dienyl)-5-hydroxy-1-(4-sulphophenyl)-1H-pyrazole-3-carboxylic acid; dipotassium 4-(5-(3-carboxy-5-oxo-1-(4-sulphophenyl)-1,5-dihydropyrazol-4-ylidene)penta-1,3-dienyl)-5-hydroxy-1-(4-sulphophenyl)-1H-pyrazole-3-carboxylic acid; 6-hydroxy-1-(2-hydroxyethyl)-5-(3-(1-(2-hydroxyethyl)-3-methyl-5-oxo-1,5-dihydropyrazol-4-ylidene)propenyl-4-methyl-1-oxo-1,2-dihydropyridine-3-carbonitrile; 6-hydroxy-5-(3-(1-(2-hydroxyethyl)-3-methyl-5-oxo-1,5-dihydropyrazol-4-ylidene)propenyl-4-methyl-2-oxo-1-(4-sulphophenyl)-1,2-dihydropyridine-3-carbonitrile; 6-hydroxy-4-methyl-5-(3-(3-methyl-5-oxo-1-(4-sulphophenyl)-1,5-dihydropyrazol-4-ylidene) propenyl)-2-oxo-1-(4-sulphophenyl)-1,2-dihydropyridine-3-carbonitrile; 6-hydroxy-4-methyl-5-(3-(3-methyl-5-oxo-1-phenyl-1,5-dihydropyrazol-4-ylidene)propenyl)-2-oxo-1-(3-sulphophenyl)-1,2-dihydropyridine-3-carbonitrile; 4-(3-(5-cyano-2-hydroxy-4-methyl-6-oxo-1-phenyl-1,6-dihydropyridin-3-yl)allylidene)-5-oxo-1-(4-sulphophenyl)-4,5-dihydro-1H-pyrazole-3-carboxylic acid; 6-hydroxy-5-(3-(1-(2-hydroxyethyl)-3-methyl-5-oxo-1,5-dihydropyrazol-4-ylidene)propenyl)-4-methyl-2-oxo-1-phenyl-1,2-dihydropyridine-3-carbonitrile; 5-(3-(4-carboxy-2,6-dioxo-1,6-dihydro-2H-pyridin-3-ylidene)propenyl)-6-hydroxy-2-oxo-1,2-dihyhdropyridine-4-carboxylic acid; 2-(3-cyano-5-(3-(5-cyano-6-dicyanomethylene-4-methyl-2-oxo-1,6-dihydro-2H-pyridin-3-ylidene)propenyl)-6-hydroxy-4-methyl-1H-pyridin-2-ylidene)malononitrile; 5-(3-(5-cyano-1-ethyl-4-methyl-2,6-dioxo-1,6-dihydro-2H-pyridin-3-ylidene)propenyl)-1-ethyl-6-hydroxy-4-methyl-2-oxo-1,2-dihydropyridine-3-carbonitrile; triethylammonium-5-(3-(5-cyano-1-ethyl-4-methyl-2,6-dioxo-1,6-dihydro-2H-pyridin-3-ylidene)propenyl)-1-ethyl-6-hydroxy-4-methyl-2-oxo-1,2-dihydropyridine-3-carbonitrile; 5-(3-(5-cyano-1-(2-hydroxyethyl)-4-methyl-2,6-dioxo-1,6-dihydro-2H-pyridin-3-ylidene)propenyl)-6-hydroxy-1-(2-hydroxyethyl)-4-methyl-2-oxo-1,2-dihydropyridine-3-carbonitrile; 5-(3-(5-cyano-1-ethyl-4-(4-methoxyphenyl)-2,6-dioxo-1,6-dihydro-2H-pyridin-3-ylidene)propenyl)-1-ethyl-6-hydroxy-4-(4-methoxyphenyl)-2-oxo-1,2-dihydropyridine-3-carbonitrile; 1-butyl-5-(3-(1-butyl-5-cyano-4-methyl-2,6-dioxo-1,6-dihydro-2H-pyridin-3-ylidene)propenyl-6-hydroxy-4-methyl-2-oxo-1,2-dihydropyridine-3-carbonitrile; 5-(3-(5-cyano-1-(3-methoxypropyl)-4-methyl-2,6-dioxo-1,6-dihydro-2H-pyridin-3-ylidene)propenyl-6-hydroxy-1-(3-hydroxypropyl)-4-methyl-2-oxo-1,2-dihydropyridine-3-carbonitrile; 5-(3-(5-cyano-1-cyclohexyl-4-methyl-2,6-dioxo-1,6-dihydro-2H-pyridin-3-ylidene)propenyl-1-ethyl-6-hydroxy-4-methyl-2-oxo-1,2-dihydropyridine-3-carbonitrile; 5-(3-(5-cyano-4-methyl-1-(2-morpholin-4-ylethyl)-2,6-dioxo-1,6-dihydro-2H-pyridin-3-ylidene)propenyl-6-hydroxy-4-methyl-1-(2-morpholin-4-ylethyl)-2-oxo-1,2-dihydropyridine-3-carbonitrile; 5-(3-(5-cyano-4-methyl-2,6-dioxo-1-(4-sulphophenyl)-1,6-dihydro-2H-pyridin-3-ylidene)-propenyl-6-hydroxy-4-methyl-2-oxo-1-(4-sulphophenyl)-1,2-dihydropyridine-3-carbonitrile; 5-(3-(5-cyano-4-methyl-2,6-dioxo-1-(3-sulphophenyl)-1,6-dihydro-2H-pyridin-3-ylidene)propenyl-6-hydroxy-4-methyl-2-oxo-1-(3-sulphophenyl)-1,2-dihydropyridine-3-carbonitrile; 5-(3-(5-cyano-1-dimethylamino-4-methyl-2,6-dioxo-1,6-dihydro-2H-pyridin-3-ylidene)propenyl-1-dimethylamino-6-hydroxy-4-methyl-2-oxo-1,2-dihydropyridine-3-carbonitrile; 5-(3-(5-carbomoyl-1-ethyl-4-methyl-2,6-dioxo-1,6-dihydro-2H-pyridin-3-ylidene)propenyl-1-ethyl-6-hydroxy-4-methyl-2-oxo-2,2-dihydropyridine-3-carboxamide; 1-ethyl-3-(3-(1-ethyl-2-hydroxy-5-methanesulphonyl-4-methyl-6-oxo-1,6-dihydropyridin-3-yl)allylidene)-5-methanesulphonyl-4-methyl-3H-pyridine-2,6-dione, 5-(5-(5-cyano-1-ethyl-4-methyl-2,6-dioxo-1,6-dihydro-2H-pyridin-3-ylidene)penta-1,3-dienyl)-1-ethyl-6-hydroxy-4-methyl-2-oxo-1,2-dihydropyridine-3-carbonitrile and ammonium 5-(3-(5-cyano-1-ethyl-4-methyl-2,6-dioxo-1,6-dihydro-2H-pyridin-3-ylidene)propenyl-1-ethyl-6-hydroxy-4-methyl-2-oxo-1,2-dihydropyridine-3-carbonitrile.

4. Agent according to one of Claims 1 to 3, **characterized in that** it comprises 0.01 to 5 percent by weight of the dyes of the formulae (I) to (III).

5. Agent according to one of Claims 1 to 4, **characterized in that** it comprises 0.01 to 15 percent by weight of at least one nonoxidative dye.

6. Agent according to one of Claims 1 to 5, **characterized in that** it has a pH of from 2 to 11.

7. Agent according to one of Claims 1 to 6, **characterized in that** it is a hair colorant.

8. Method for the semipermanent colouring of hair, **characterized in that** 50 to 150 grams of an agent according to one of Claims 1 to 7 are applied to the hair, and the hair is rinsed with water after a contact time of from 10 to 45 minutes at 20 to 50°C, optionally washed with a shampoo and/or after-rinsed with an aqueous solution of a weak organic acid, such as, for example, citric acid or tartaric acid, and then dried.

## Revendications

1. Composition pour la teinture de fibres de kératine, **caractérisée en ce qu'**elle contient une association de (a) au moins un colorant non oxydant, naturel ou synthétique, qui est choisi parmi des colorants naturels ou des colorants nitrés, des colorants azoïques, des colorants quinoniques, des colorants triphénylméthane et des colorants basiques ou acides, et (b) au moins un colorant polyméthine de formules générales (I) à (III), ou ses sels physiologiquement acceptables,

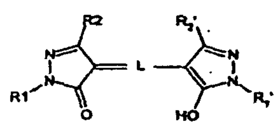

(I)

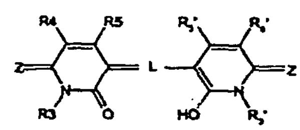

(II)

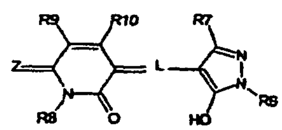

(III)

formules dans lesquelles **R1, R1'** et **R6** représentent un atome d'hydrogène, un groupe alkyle en $C_1$-$C_8$ à chaîne droite ou ramifiée, un groupe hydroxyéthyle, un groupe dihydroxypropyle, un groupe méthoxyéthyle, un groupe carboxyéthyle, un groupe sulfoalkyle en $C_1$-$C_4$, un radical phényle ou un radical phényle substitué par un ou plusieurs atomes d'halogène, un ou deux groupes sulfo, un ou deux groupes carboxy, un ou plusieurs groupes alkyle en $C_1$-$C_8$ ou alcoxy en $C_1$-$C_8$, ramifiés ou non ramifiés, un radical benzyle ou un radical benzyle substitué par un ou plusieurs atomes d'halogène, par un groupe alkyle en $C_1$-$C_4$, un groupe hydroxy, un groupe méthoxy, un groupe carboxy, un groupe nitro ou un groupe amino, ou représentent un hétérocycle à 5 chaînons ou 6 chaînons, saturé ou insaturé, **R1** et **R1'** pouvant être identiques ou différents, et
**R2, R2'** et **R7** représentent un atome d'hydrogène, un groupe alkyle en $C_1$-$C_6$ ramifié ou non ramifié, un radical phényle, un groupe amino qui peut être additionnellement acylé ou sulfonylé, un groupe acétyle, un groupe méthoxy, un groupe carboxy qui peut être estérifié par un alcool en $C_1$-$C_8$ à chaîne droite ou ramifiée ou par un éther monométhylique d'éthylèneglycol ou par un éther monoéthylique d'éthylèneglycol, un groupe carboxamido, un groupe carboxanilide, un groupe 2-amino-2-oxyéthyle ou un groupe nitrile, **R2** et **R2'** pouvant être identiques ou différents, et

**R3, R3'** et **R8** représentent un atome d'hydrogène, un groupe alkyle en $C_1$-$C_{11}$ à chaîne droite ou ramifiée, un groupe monohydroxyalkyle en $C_1$-$C_{11}$ à chaîne droite ou ramifiée, un groupe dihydroxyalkyle en $C_1$-$C_{11}$ à chaîne droite ou ramifiée, un groupe alcoxyalkyle en $C_1$-$C_{11}$ à chaîne droite ou ramifiée, un groupe monoalkylamino en $C_1$-$C_{11}$ à chaîne droite ou ramifiée, un groupe amino de formule $(CH_2)_x$-NR11R12 (où x représente un nombre entier allant de 0 à 3 et R11 et R12 représentent, indépendamment l'un de l'autre, un groupe alkyle en $C_1$-$C_3$), un groupe carboxyalkyle ou sulfoalkyle en $C_2$-$C_4$, un groupe phényle ou un groupe phényle substitué par un ou plusieurs atomes d'halogène, un ou deux groupes sulfo, un ou deux groupes carboxy, un ou plusieurs groupes alkyle en $C_1$-$C_8$ ou alcoxy en $C_1$-$C_8$ ramifiés ou non ramifiés, un groupe benzyle ou un groupe benzyle substitué par un ou plusieurs atomes d'halogène, par un groupe alkyle en $C_1$-$C_4$, un groupe hydroxy, un groupe méthoxy, un groupe nitro ou un groupe amino, ou representent un groupe phényléthyle, un hétérocycle aromatique ou non aromatique à 5 chaînons ou 6 chaînons, qui peut être lié directement ou par l'intermédiaire d'un groupe méthylène, un groupe pyrrolidino, un groupe morpholino, un groupe pipérazino, un groupe pipéridino ou un groupe pyridino-alkyle($C_2$ ou $C_3$) ou un groupe trialkylammoniumalkyle de formule $R13\text{-}N(R14)_3^+$ (où R13 représente un groupe alkylène en $C_1$-$C_6$ et R14 représente un groupe méthyle ou éthyle, le nombre total d'atomes de carbone dans la molécule allant de 5 à 9), **R3** et **R3'** pouvant être identiques ou différents, et

**R4, R4'** et **R9** représentent un atome d'hydrogène, un groupe nitrile, un groupe ester d'acide carboxylique, un groupe carboxamido, un groupe sulfo, un groupe sulfométhyle, un groupe méthanesulfonyle, un radical pyridinium ou un radical imidazolium, **R4** et **R4'** pouvant être identiques ou différents, et

**R5, R5'** et **R10** représentent un atome d'hydrogène, un groupe alkyle en $C_1$-$C_4$, un groupe cycloalkyle en $C_5$-$C_6$, un groupe phényle, un groupe méthoxyphényle, un groupe benzyle, un groupe phényléthyle ou un groupe carboxy, **R5** et **R5'** pouvant être identiques ou différents, et

**Z** représente un atome d'oxygène ou un radical de formule $C(CN)_2$, C(CN)COOQ ou $C(COOQ)_2$, où Q représente dans chaque cas un groupe alkyle en $C_1$-$C_8$ ou un monoalkyl($C_3$-$C_7$)éther d'éthylèneglycol, et

**L** représente un groupe pontant de formule générale

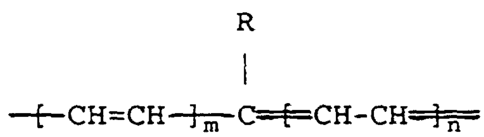

dans laquelle **R** représente un atome d'hydrogène, un groupe phényle, un groupe méthyle, un groupe carbamoyle ou un atome d'halogène,

et les indices **m** et **n** peuvent être chacun 0, 1 ou 2, la somme de **m** et **n** n'étant pas supérieure à 2.

**2.** Composition selon la revendication 1, **caractérisée en ce que** le sel physiologiquement acceptable est choisi parmi les sels d'ammonium, les sels de sodium, les sels de potassium, les sels de triéthylammonium, les sels de N-méthylmorpholinium, les sels de monoéthanolammonium, les sels de diéthanolammonium et les sels de triéthanolammonium.

**3.** Composition selon la revendication 1, **caractérisée en ce que** les colorants de formules (I) à (III) sont choisis parmi la 4-(5-hydroxy-1,3-diméthyl-1H-pyrazol-4-ylméthylène)-2,5-diméthyl-2,4-dihydropyrazol-3-one, la 2-(2-hydroxyéthyl)-4-(5-hydroxy-1-(2-hydroxyéthyl)-3-méthyl-1H-pyrazol-4-ylméthylène)-5-méthyl-2,4-dihydropyrazol-3-one, la 4-(5-hydroxy-3-méthyl-1-(4-sulfophényl)-1H-pyrazol-4-ylméthylène)-5-méthyl-2-(4-sulfophényl)-2,4-dihydropyrazol-3-one, la diammonium-4-(5-hydroxy-3-méthyl-1-(4-sulfophényl)-1H-pyrazol-4-ylméthylène)-5-méthyl-2-(4-sulfophényl)-2,4-dihydropyrazol-3-one, la 4-(3-(5-hydroxy-1,3-diméthyl-1H-pyrazol-4-yl)allylidène)-2,5-diméthyl-2,4-dihydropyrazol-3-one, la 5-amino-4-(3-(3-amino-5-hydroxy-1-phényl-1H-pyrazol-4-yl)allylidène)-2-phényl-2,4-dihydropyrazol-3-one, la 2-(2-hydroxyéthyl)-4-(3-(5-hydroxy-1-(2-hydroxyéthyl)-3-méthyl-1H-pyrazol-4-yl)-allylidène)-5-méthyl-2,4-dihydropyrazol-3-one, la 4-(3-(5-hydroxy-3-méthyl-1-(4-sulfophényl)-1H-pyrazol-4-yl)-allylidène)-5-méthyl-2-(4-sulfophényl)-2,4-dihydropyrazol-3-one, la disodium-4-(3-(5-hydroxy-3-méthyl-1-(4-sulfophényl)-1H-pyrazol-4-yl)allylidène)-5-méthyl-2-(4-sulfophényl)-2,4-dihydropyrazol-3-one, la 4-(3-(5-hydroxy-3-méthyl-1-(4-sulfophényl)-1H-pyrazol-4-yl)but-2-énylidène)-5-méthyl-2-(4-sulfophényl)-2,4-dihydropyrazol-3-one, la tripotassium-4-(3-(5-hydroxy-3-méthyl-1-(4-sulfophényl)-1H-pyrazol-4-yl)but-2-énylidène)-5-méthyl-2-(4-sulfophényl)-2,4-dihydropyrazol-3-one, l'acide 4-(3-(3-carboxy-5-oxo-1-(4-sulfophényl)-1,5-dihydropyrazol-4-ylidène)propényl)-5-hydroxy-1-(4-sulfophényl)-1H-pyrazole-3-carboxylique, le 4-(3-(3-carboxy-5-oxo-1-(4-sulfophényl)-1,5-dihydropyrazol-4-ylidène)propényl)-5-hydroxy-1-(4-sulfophényl)-1H-pyrazole-3-carboxylate tétrapotassique, le 5-hydroxy-4-(3-(3-méthoxycarbonyl-5-oxo-1-(4-sulfophényl)-1,5-dihydropyrazol-4-ylidène)propényl)-1-(4-sulfophényl)-1H-pyrazole-3-carboxylate de méthyle, la 4-(5-(5-hydroxy-1,3-diméthyl-1H-pyrazol-4-yl)penta-2,4-diénylidène)-2,5-diméthyl-2,4-dihydropyrazol-3-one, la 5-amino-4-(5-(3-amino-5-hydroxy-1-phényl-1H-pyrazol-4-yl)penta-2,4-diénylidène)-2-phényl-2,4-dihydropyrazol-3-one, la 2-(2-hydroxyéthyl)-4-(5-(5-hydroxy-1-(2-hydroxyéthyl)-3-méthyl-1H-pyrazol-4-yl)penta-2,4-diénylidène)-5-méthyl-2,4-dihydropyrazol-3-one, la 4-(5-(5-hydroxy-3-méthyl-1-(4-sulfophényl)-1H-pyrazol-4-yl)penta-2,4-diénylidène)-5-méthyl-2-(4-sulfophényl)-2,4-dihydropyrazol-3-one, la dipotassium-4-(5-(5-hydroxy-3-méthyl-1-(4-sulfophényl)-1H-pyrazol-4-yl)penta-2,4-diénylidène)-5-méthyl-2-(4-sulfophényl)-2,4-dihydropyrazol-3-one, l'acide 4-(5-(3-carboxy-5-oxo-1-(4-sulfophényl)-1,5-dihydropyrazol-4-ylidène)-penta-1,3-diényl)-5-hydroxy-1-(4-sulfophényl)-1H-pyrazole-3-carboxylique, le 4-(5-(3-carboxy-5-oxo-1-(4-sulfophényl)-1,5-dihydropyrazol-4-ylidène)penta-1,3-diényl)-5-hydroxy-1-(4-sulfophényl)-1H-pyrazole-3-carboxylate dipotassique, le 6-hydroxy-1-(2-hydroxyéthyl)-5-(3-(1-(2-hydroxyéthyl)-3-méthyl-5-oxo-1,5-dihydropyrazol-4-ylidène)propényl)-4-méthyl-2-oxo-1,2-dihydropyridine-3-carbonitrile, le 6-hydroxy-5-(3-(1-(2-hydroxyéthyl)-3-méthyl-5-oxo-1,5-dihydropyrazol-4-ylidène)propényl)-4-méthyl-2-oxo-1-(4-sulfophényl)-1,2-dihydropyridine-3-carbonitrile, le 6-hydroxy-4-méthyl-5-(3-(3-méthyl-5-oxo-1-(4-sulfophényl)-1,5-dihydropyrazol-4-ylidène)propényl)-2-oxo-1-(4-sulfophényl)-1,2-dihydropyridine-3-carbonitrile, le 6-hydroxy-4-méthyl-5-(3-(3-méthyl-5-oxo-1-phényl-1,5-dihydropyrazol-4-ylidène)propényl)-2-oxo-1-(3-sulfophényl)-1,2-dihydropyridine-3-carbonitrile, l'acide 4-(3-(5-cyano-2-hydroxy-4-méthyl-6-oxo-1-phényl-1,6-dihydropyridin-3-yl)allylidène)-5-oxo-1-(4-sulfophényl)-4,5-dihydro-1H-pyrazole-3-carboxylique, le 6-hydroxy-5-(3-(1-(2-hydroxyéthyl)-3-méthyl-5-oxo-1,5-dihydropyrazol-4-ylidène)propényl)-4-méthyl-2-oxo-1-phényl-1,2-dihydropyridine-3-carbonitrile, l'acide 5-(3-(4-carboxy-2,6-dioxo-1,6-dihydro-2H-pyridin-3-ylidène)propényl)-6-hydroxy-2-oxo-1,2-dihydropyridine-4-carboxylique, le 2-(3-cyano-5-(3-(5-cyano-6-dicyanométhylène-4-méthyl-2-oxo-1,6-dihydro-2H-pyridin-3-ylidène)propényl)-6-hydroxy-4-méthyl-1H-pyridin-2-ylidène)malononitrile, le 5-(3-(5-cyano-1-éthyl-4-méthyl-2,6-dioxo-1,6-dihydro-2H-pyridin-3-ylidène)-propényl)-1-éthyl-6-hydroxy-4-méthyl-2-oxo-1,2-dihydropyridine-3-carbonitrile, le triéthylammonium-5-(3-(5-cyano-1-éthyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carbonitrile, le 5-(3-(5-cyano-1-(2-hydroxyéthyl)-4-méthyl-2,6-dioxo-1,6-dihydro-2H-pyridin-3-ylidène)propényl)-6-hydroxy-1-(2-hydroxyéthyl)-4-méthyl-2-oxo-1,2-dihydropyridine-3-carbonitrile, le 5-(3-(5-cyano-1-éthyl-4-(4-méthoxyphényl)-2,6-dioxo-1,6-dihydro-2H-pyridin-3-ylidène)-propényl)-1-éthyl-6-hydroxy-4-(4-méthoxyphényl)-2-oxo-1,2-dihydropyridine-3-carbonitrile, le 1-butyl-5-(3-(1-butyl-5-cyano-4-méthyl-2,6-dioxo-1,6-dihydro-2H-pyridin-3-ylidène)propényl)-6-hydroxy-4-méthyl-2-oxo-1,2-dihydropyridine-3-carbonitrile, le 5-(3-(5-cyano-1-(3-méthoxypropyl)-4-méthyl-2,6-dioxo-1,6-dihydro-2H-pyridin-3-ylidène)propényl)-6-hydroxy-1-(3-méthoxypropyl)-4-méthyl-2-oxo-1,2-dihydropyridine-3-carbonitrile, le 5-(3-(5-cyano-1-cyclohexyl-4-méthyl-2,6-dioxo-1,6-dihydro-2H-pyridin-3-ylidène)propényl)-1-éthyl-6-hydroxy-4-méthyl-2-oxo-1,2-dihydropyridine-3-carbonitrile, le 5-(3-(5-cyano-4-méthyl-1-(2-morpholin-4-yléthyl)-2,6-dioxo-1,6-dihydro-2H-pyridin-3-ylidène)-propényl)-6-hydroxy-4-méthyl-1-(2-morpholin-4-yléthyl)-2-oxo-1,2-dihydropyridine-3-carbonitrile, le 5-(3-(5-cyano-4-méthyl-2,6-dioxo-1-(4-sulfophényl)-1,6-dihydro-2H-pyridin-3-ylidène)propényl)-6-hydroxy-4-méthyl-2-oxo-1-(4-sulfophényl)-1,2-dihydropyridine-3-carbonitrile, le 5-(3-(5-cyano-4-méthyl-2,6-dioxo-1-(3-sulfophényl)-1,6-dihydro-2H-pyridin-3-ylidène)-propényl)-6-hydroxy-4-méthyl-2-oxo-1-(3-sulfophényl)-1,2-dihydropyridine-3-carbonitrile, le 5-(3-(5-cyano-1-diméthylamino-4-méthyl-2,6-dioxo-1,6-dihydro-2H-pyridin-3-ylidène)propényl)-1-diméthylamino-6-hydroxy-4-méthyl-2-oxo-1,2-dihydropyridine-3-carbonitrile, le 5-(3-(5-carbamoyl-1-éthyl-4-méthyl-2,6-dioxo-1,6-dihydro-2H-pyridin-3-ylidène)propényl)-1-éthyl-6- ' hydroxy-4-méthyl-2-oxo-1,2-dihydropyridine-3-carboxamide, la 1-éthyl-3-(3-(1-éthyl-2-hydroxy-5-méthanesulfonyl-4-méthyl-6-oxo-1,6-dihydropyridin-3-yl)allylidène)-5-méthanesulfonyl-4-méthyl-3H-pyridine-2,6-dione, le 5-(5-(5-cyano-1-éthyl-4-méthyl-2,6-dioxo-1,6-dihydro-2H-pyridin-3-ylidène)penta-1,3-diényl)-1-éthyl-6-hydroxy-4-méthyl-2-oxo-1,2-dihydropyridine-3-carbonitrile et l'ammonium-5-(3-(5-cyano-1-éthyl-4-méthyl-2,6-dioxo-1,6-dihydro-2H-pyridin-3-ylidène)-propényl)-1-éthyl-6-hydroxy-4-méthyl-2-oxo-1,2-dihydropyridine-3-carbonitrile.

4. Composition selon l'une quelconque des revendications 1 à 3, **caractérisée en ce qu'**elle contient de 0,01 à 5 % en poids des colorants de formules (I) à (III).

5. Composition selon l'une quelconque des revendications 1 à 4, **caractérisée en ce qu'**elle contient de 0,01 à 15 % en poids d'au moins un colorant non oxydant.

6. Composition selon l'une quelconque des revendications 1 à 5, **caractérisée en ce qu'**elle présente un pH de 2 à 11.

7. Composition selon l'une quelconque des revendications 1 à 6, **caractérisée en ce qu'**il s'agit d'une composition de teinture pour cheveux.

8. Procédé pour la teinture semi-permanente des cheveux, **caractérisé en ce qu'**on applique sur les cheveux 50 à

150 grammes d'une composition selon l'une quelconque des revendications 1 à 7 et, après un temps d'action de 10 à 45 minutes à 20-50°C, on rince à l'eau les cheveux, éventuellement on les lave avec un shampooing et/ou on les soumet à un après-rinçage avec la solution aqueuse d'un acide organique faible, comme par exemple l'acide citrique ou l'acide tartrique, et ensuite on les sèche.